# EUROPEAN PATENT APPLICATION

(11) **EP 2 230 246 A1**
(43) Date of publication of application: **22.09.2010**
(21) Application number: 09003958.7
(22) Date of filing: 19.03.2009
(51) Int. Cl.: C07K 14/08, A61K 39/12

(54) **Arterivirus glycoprotein 5 virus-like particles**

(71) Applicant: Fraunhofer-Gesellschaft zur Förderung der angewandten Forschung e.V., 80686 München (DE)
(72) Inventor: Giese, Matthias, Dr., 69123 Heidelberg (DE); Ulbert, Sebastian, Dr., 04275 Leipzig (DE)
(74) Representative: Grund, Martin

(57) **Abstract**

The present invention provides a method for the production of a virus-like particle comprising an arterivirus full-length GP₅ protein and/or an arterivirus GP₅ protein with a modified C-terminus or a functional homologue thereof. The method further comprises transfecting the mammalian cell with a nucleic acid coding for said GP₅ protein, upon which the cell will secrete virus-like particles into a cell culturing medium. Such virus-like particles can be effectively used for vaccination purposes.

SEQUENCE LISTING

<110> Fraunhofer-Gesellschaft zur Förderung der angewandten Forschung e.V.

<120> Arterivirus glycoprotein 5 virus-like particles

<130> 144-007

<160> 18

<170> PatentIn version 3.3

<210> 1
<211> 201
<212> PRT
<213> Porcine reproductive and respiratory syndrome virus

<400> 1

Met Arg Cys Ser His Lys Leu Gly Arg Phe Leu Thr Pro His Ser Cys
1               5                   10                  15

Phe Trp Trp Leu Phe Leu Leu Cys Thr Gly Leu Ser Trp Ser Phe Ala
            20                  25                  30

Asp Gly Asn Gly Asp Ser Ser Thr Tyr Gln Tyr Ile Tyr Asn Leu Thr
        35                  40                  45

Ile Cys Glu Leu Asn Gly Thr Asp Trp Leu Ser Ser His Phe Gly Trp
    50                  55                  60

Ala Val Glu Thr Phe Val Leu Tyr Pro Val Ala Thr His Ile Leu Ser
65                  70                  75                  80

Leu Gly Phe Leu Thr Thr Ser His Phe Phe Asp Ala Leu Gly Leu Gly
                85                  90                  95

Ala Val Ser Thr Ala Gly Phe Val Gly Gly Arg Tyr Val Leu Cys Ser
            100                 105                 110

Val Tyr Gly Ala Cys Ala Phe Ala Ala Phe Val Cys Phe Val Ile Arg
        115                 120                 125

Ala Ala Lys Asn Cys Met Ala Cys Arg Tyr Ala Arg Thr Arg Phe Thr
    130                 135                 140

Asn Phe Ile Val Asp Asp Arg Gly Arg Val His Arg Trp Lys Ser Pro
145                 150                 155                 160

Ile Val Val Glu Lys Leu Gly Lys Ala Glu Val Asp Gly Asn Leu Val
                165                 170                 175

Thr Ile Lys His Val Val Leu Glu Gly Val Lys Ala Gln Pro Leu Thr
            180                 185                 190

Arg Thr Ser Ala Glu Gln Trp Glu Ala
        195                 200

<210>  2
<211>  200
<212>  PRT
<213>  Porcine reproductive and respiratory syndrome virus

<400>  2

Met Leu Glu Lys Cys Leu Thr Ala Gly Cys Tyr Ser Gln Leu Leu Ser
1               5                   10                  15

Leu Trp Cys Ile Val Pro Phe Cys Phe Ala Val Leu Val Asn Ala Ser
            20                  25                  30

Asn Asp Ser Ser Ser His Leu Gln Leu Ile Tyr Asn Leu Thr Leu Cys
        35                  40                  45

Glu Leu Asn Gly Thr Asp Trp Leu Ala Asn Lys Phe Asp Trp Ala Val
    50                  55                  60

Glu Ser Phe Val Ile Phe Pro Val Leu Thr His Ile Val Ser Tyr Gly
65                  70                  75                  80

Ala Leu Thr Thr Ser His Phe Leu Asp Thr Val Ala Leu Val Thr Val
                85                  90                  95

Ser Thr Ala Gly Phe Val His Gly Arg Tyr Val Leu Ser Ser Ile Tyr
            100                 105                 110

Ala Val Cys Ala Leu Ala Ala Leu Thr Cys Phe Val Ile Arg Phe Ala
        115                 120                 125

Lys Asn Cys Met Ser Trp Arg Tyr Ala Cys Thr Arg Tyr Thr Asn Phe
    130                 135                 140

Leu Leu Asp Thr Lys Gly Arg Leu Tyr Arg Trp Arg Ser Pro Val Ile
145                 150                 155                 160

Ile Glu Lys Arg Gly Lys Val Glu Val Glu Gly His Leu Ile Asp Leu
                165                 170                 175

Lys Arg Val Val Leu Asp Gly Ser Val Ala Thr Pro Ile Thr Arg Val
            180                 185                 190

Ser Ala Glu Gln Trp Gly Arg Pro
        195                 200

<210>  3
<211>  255
<212>  PRT
<213>  Equine arteritis virus

<400>  3

Met Leu Ser Met Ile Val Leu Leu Phe Leu Leu Trp Gly Ala Pro Ser
1               5                   10                  15

His Ala Tyr Phe Ser Tyr Tyr Thr Ala Gln Arg Phe Thr Asp Phe Thr
            20                  25                  30

Leu Cys Met Leu Thr Asp Arg Gly Val Ile Ala Asn Leu Leu Arg Tyr
        35                  40                  45

Asp Glu His Thr Ala Leu Tyr Asn Cys Ser Ala Ser Lys Thr Cys Trp
    50                  55                  60

Tyr Cys Thr Phe Leu Asp Glu Gln Ile Ile Thr Phe Gly Thr Asp Cys
65                  70                  75                  80

Asn Asp Thr Tyr Ala Val Pro Val Ala Glu Val Leu Glu Gln Ala His
                85                  90                  95

Gly Pro Tyr Ser Val Leu Phe Asn Asp Met Pro Pro Phe Ile Tyr Tyr
            100                 105                 110

Gly Arg Glu Phe Gly Ile Val Val Leu Asp Val Phe Met Phe Tyr Pro
        115                 120                 125

Val Leu Val Leu Phe Phe Leu Ser Val Leu Pro Tyr Ala Thr Leu Ile
    130                 135                 140

Leu Glu Met Cys Val Ser Ile Leu Phe Ile Ile Tyr Gly Ile Tyr Ser
145                 150                 155                 160

Gly Ala Tyr Leu Ala Met Gly Ile Phe Ala Ala Thr Leu Ala Ile His
                165                 170                 175

Ser Ile Val Val Leu Arg Gln Leu Leu Trp Leu Cys Leu Ala Trp Arg
            180                 185                 190

Tyr Arg Cys Thr Leu His Ala Ser Phe Ile Ser Ala Glu Gly Lys Val
        195                 200                 205

Tyr Pro Val Asp Pro Gly Leu Pro Val Ala Ala Ala Gly Asn Arg Leu
    210                 215                 220

Leu Val Pro Gly Arg Pro Thr Ile Asp Tyr Ala Val Ala Tyr Gly Ser
225                 230                 235                 240

Lys Val Asn Leu Val Arg Leu Gly Ala Ala Glu Val Trp Glu Pro
                245                 250                 255

<210>  4
<211>  211
<212>  PRT
<213>  Artificial

<220>
<223>  PRRSV lelystad GP5 fused to HA-tag

<400>  4

Met Arg Cys Ser His Lys Leu Gly Arg Phe Leu Thr Pro His Ser Cys
1               5                   10                  15

Phe Trp Trp Phe Phe Leu Leu Cys Thr Gly Leu Ser Trp Ser Phe Ala
            20                  25                  30

Asp Gly Asn Gly Asn Ser Ser Thr Tyr Gln Tyr Ile Tyr Asn Leu Thr
        35                  40                  45

Ile Cys Glu Leu Asn Gly Thr Asp Trp Leu Ser Ser His Phe Gly Trp
    50                  55                  60

Ala Val Glu Thr Phe Val Phe Tyr Pro Val Ala Thr His Ile Leu Ser
65                  70                  75                  80

Leu Gly Phe Leu Thr Thr Ser His Phe Phe Asp Ala Leu Gly Leu Gly
                85                  90                  95

Ala Val Ser Thr Ala Gly Phe Val Gly Gly Arg Tyr Val Leu Cys Ser
            100                 105                 110

Val Tyr Gly Ala Cys Ala Phe Ala Ala Phe Val Cys Phe Val Ile Arg
        115                 120                 125

Ala Ala Lys Asn Cys Met Ala Cys Arg Tyr Ala Arg Thr Arg Phe Thr
    130                 135                 140

Asn Phe Ile Val Asp Asn Arg Gly Arg Val His Arg Trp Lys Ser Pro
145                 150                 155                 160

Ile Val Val Glu Lys Leu Gly Lys Ala Glu Val Asp Gly Asn Leu Val

165                 170                 175

Thr Ile Lys His Val Val Leu Glu Gly Val Lys Ala Gln Pro Leu Thr
            180                 185                 190

Arg Thr Ser Ala Glu Gln Trp Glu Ala Ala Tyr Pro Tyr Asp Val Pro
        195                 200                 205

Asp Tyr Ala
    210

<210>  5
<211>  210
<212>  PRT
<213>  artificial

<220>
<223>  PRRSV VR-2332 GP5 fused to HA-tag

<400>  5

Met Leu Glu Lys Cys Leu Thr Ala Gly Cys Cys Ser Arg Leu Leu Ser
1               5                   10                  15

Leu Trp Cys Ile Val Pro Phe Cys Phe Ala Val Leu Ala Asn Ala Ser
            20                  25                  30

Asn Asp Ser Ser Ser His Leu Gln Leu Ile Tyr Asn Leu Thr Leu Cys
        35                  40                  45

Glu Leu Asn Gly Thr Asp Trp Leu Ala Asn Lys Phe Asp Trp Ala Val
    50                  55                  60

Glu Ser Phe Val Ile Phe Pro Val Leu Thr His Ile Val Ser Tyr Gly
65                  70                  75                  80

Ala Leu Thr Thr Ser His Phe Leu Asp Thr Val Ala Leu Val Thr Val
                85                  90                  95

Ser Thr Ala Gly Phe Val His Gly Arg Tyr Val Leu Ser Ser Ile Tyr
            100                 105                 110

Ala Val Cys Ala Leu Ala Ala Leu Thr Cys Phe Val Ile Arg Phe Ala
        115                 120                 125

Lys Asn Cys Met Ser Trp Arg Tyr Ala Cys Thr Arg Tyr Thr Asn Phe
    130                 135                 140

Leu Leu Asp Thr Lys Gly Arg Leu Tyr Arg Trp Arg Ser Pro Val Ile
145                 150                 155                 160

Ile Glu Lys Arg Gly Lys Val Glu Val Glu Gly His Leu Ile Asp Leu
                165                 170                 175

Lys Arg Val Val Leu Asp Gly Ser Val Ala Thr Pro Ile Thr Arg Val
            180                 185                 190

Ser Ala Glu Gln Trp Gly Arg Pro Ala Tyr Pro Tyr Asp Val Pro Asp
        195                 200                 205

Tyr Ala
    210

<210>  6
<211>  265
<212>  PRT
<213>  artificial

<220>
<223>  EAV GP5 fused to HA-tag

<400>  6

Met Leu Ser Met Ile Val Leu Leu Phe Leu Leu Trp Gly Ala Pro Ser
1               5                   10                  15

His Ala Tyr Phe Ser Tyr Tyr Thr Ala Gln Arg Phe Thr Asp Phe Thr
            20                  25                  30

Leu Cys Met Leu Thr Asp Arg Gly Val Ile Ala Asn Leu Leu Arg Tyr
        35                  40                  45

Asp Glu His Thr Ala Leu Tyr Asn Cys Ser Ala Ser Lys Thr Cys Trp
    50                  55                  60

Tyr Cys Thr Phe Leu Asp Glu Gln Ile Ile Thr Phe Gly Thr Asp Cys
65                  70                  75                  80

Asn Asp Thr Tyr Ala Val Pro Val Ala Glu Val Leu Glu Gln Ala His
                85                  90                  95

Gly Pro Tyr Ser Val Leu Phe Asn Asp Met Pro Pro Phe Ile Tyr Tyr
            100                 105                 110

Gly Arg Glu Phe Gly Ile Val Val Leu Asp Val Phe Met Phe Tyr Pro
        115                 120                 125

Val Leu Val Leu Phe Phe Leu Ser Val Leu Pro Tyr Ala Thr Leu Ile
    130                 135                 140

Leu Glu Met Cys Val Ser Ile Leu Phe Ile Ile Tyr Gly Ile Tyr Ser
145                 150                 155                 160

Gly Ala Tyr Leu Ala Met Gly Ile Phe Ala Ala Thr Leu Ala Ile His
                165                 170                 175

Ser Ile Val Val Leu Arg Gln Leu Leu Trp Leu Cys Leu Ala Trp Arg
            180                 185                 190

Tyr Arg Cys Thr Leu His Ala Ser Phe Ile Ser Ala Glu Gly Lys Val
        195                 200                 205

Tyr Pro Val Asp Pro Gly Leu Pro Val Ala Ala Ala Gly Asn Arg Leu
    210                 215                 220

Leu Val Pro Gly Arg Pro Thr Ile Asp Tyr Ala Val Ala Tyr Gly Ser
225                 230                 235                 240

Lys Val Asn Leu Val Arg Leu Gly Ala Ala Glu Val Trp Glu Pro Ala
                245                 250                 255

Tyr Pro Tyr Asp Val Pro Asp Tyr Ala
            260                 265

<210>  7
<211>  5634
<212>  DNA
<213>  Artificial

<220>
<223>  Plasmid GP5_lely_pT; BamH I / Xho I; Cloning of GP5 PRRSV
       Lelystad into pCR3.1

<220>
<221>  misc_feature
<222>  (682)..(1332)
<223>  BamH I - Xho I ; PRRSV lelystad GP5

<400>  7
gcgcgcgttg acattgatta ttgactagtt attaatagta atcaattacg gggtcattag      60

ttcatagccc atatatggag ttccgcgtta cataacttac ggtaaatggc ccgcctggct     120

gaccgcccaa cgacccccgc ccattgacgt caataatgac gtatgttccc atagtaacgc     180

caatagggac tttccattga cgtcaatggg tggactattt acggtaaact gcccacttgg     240

cagtacatca agtgtatcat atgccaagta cgccccctat tgacgtcaat gacggtaaat     300

ggcccgcctg gcattatgcc cagtacatga ccttatggga ctttcctact tggcagtaca     360

tctacgtatt agtcatcgct attaccatgg tgatgcggtt ttggcagtac atcaatgggc     420

gtggatagcg gtttgactca cggggatttc caagtctcca ccccattgac gtcaatggga 480
gtttgttttg gcaccaaaat caacgggact ttccaaaatg tcgtaacaac tccgccccat 540
tgacgcaaat gggcggtagg cgtgtacggt gggaggtcta tataagcaga gctctctggc 600
taactagaga acccactgct tactggctta tcgaaattaa tacgactcac tatagggaga 660
cccaagcttg gtaccgagct cggatccacc atgagatgtt ctcacaaatt ggggcgtttc 720
ttgactccgc actcttgctt ctggtggctt tttttgctgt gtaccggttt gtcctggtcc 780
tttgccgatg gcaacggcaa cagctcgaca taccaataca tatataactt gacgatatgc 840
gagctgaatg ggaccgactg gttgtccagc cattttggtt gggcagtcga gacctttgtg 900
ttttacccgg ttgccactca tatcctctca ctgggttttc tcacaacaag ccattttttt 960
gacgcgctcg gtctcggcgc tgtatccact gcaggatttg ttggcgggcg gtatgtactc 1020
tgcagcgtct acggcgcttg tgctttcgca gcgttcgtat gttttgtcat ccgtgctgct 1080
aaaaattgca tggcctgccg ctatgcccgt acccggttta ccaacttcat tgtagacaac 1140
cgggggagag ttcatcgatg gaagtctcca atagtggtag aaaaattggg caaagccgaa 1200
gtcgacggca acctcgtcac catcaaacat gtcgtcctcg aaggggttaa agctcaaccc 1260
ttgacgagga cttcggctga gcaatgggag gcctagacga tttttgcaac gaaaaaaaaa 1320
aaaaaaaaaa actcgagcat gcatctagag ggccctattc tatagtgtca cctaaatgct 1380
agagctcgct gatcagcctc gactgtgcct tctagttgcc agccatctgt tgtttgcccc 1440
tcccccgtgc cttccttgac cctggaaggt gccactccca ctgtcctttc ctaataaaat 1500
gaggaaattg catcgcattg tctgagtagg tgtcattcta ttctgggggg tggggtgggg 1560
caggacagca agggggagga ttgggaagac aatagcaggc atgctgggga tgcggtgggc 1620
tctatggctt ctgaggcgga aagaaccagt ggcggtaata cggttatcca cagaatcagg 1680
ggataacgca ggaaagaaca tgtgagcaaa aggccagcaa aaggccagga accgtaaaaa 1740
ggccgcgttg ctggcgtttt tccataggct ccgcccccct gacgagcatc acaaaaatcg 1800
acgctcaagt cagaggtggc gaaacccgac aggactataa agataccagg cgtttccccc 1860
tggaagctcc ctcgtgcgct ctcctgttcc gaccctgccg cttaccggat acctgtccgc 1920
ctttctccct tcgggaagcg tggcgctttc tcatagctca cgctgtaggt atctcagttc 1980
ggtgtaggtc gttcgctcca agctgggctg tgtgcacgaa ccccccgttc agcccgaccg 2040
ctgcgcctta tccggtaact atcgtcttga gtccaacccg gtaagacacg acttatcgcc 2100
actggcagca gccactggta acaggattag cagagcgagg tatgtaggcg gtgctacaga 2160
gttcttgaag tggtggccta actacggcta cactagaagg acagtatttg gtatctgcgc 2220
tctgctgaag ccagttacct tcggaaaaag agttggtagc tcttgatccg gcaaacaaac 2280
caccgctggt agcggtggtt tttttgtttg caagcagcag attacgcgca gaaaaaaagg 2340

atctcaagaa gatcctttga tcttttctac ggggtctgac gctcagtgga acgaaaactc 2400

acgttaaggg attttggtca tgagattatc aaaaaggatc ttcacctaga tccttttaaa 2460

ttaaaaatga agttttaaat caatctaaag tatatatgag taacctgagg ctatggcagg 2520

gcctgccgcc ccgacgttgg ctgcgagccc tgggccttca cccgaacttg gggggtgggg 2580

tggggaaaag gaagaaacgc gggcgtattg gccccaatgg ggtctcggtg gggtatcgac 2640

agagtgccag ccctgggacc gaaccccgcg tttatgaaca aacgacccaa caccgtgcgt 2700

tttattctgt ctttttattg ccgtcatagc gcgggttcct tccggtattg tctccttccg 2760

tgtttcagtt agcctccccc tagggtgggc gaagaactcc agcatgagat ccccgcgctg 2820

gaggatcatc cagccggcgt cccggaaaac gattccgaag cccaaccttt catagaaggc 2880

ggcggtggaa tcgaaatctc gtgatggcag gttgggcgtc gcttggtcgg tcatttcgaa 2940

ccccagagtc ccgctcagaa gaactcgtca agaaggcgat agaaggcgat gcgctgcgaa 3000

tcgggagcgg cgataccgta aagcacgagg aagcggtcag cccattcgcc gccaagctct 3060

tcagcaatat cacgggtagc caacgctatg tcctgatagc ggtccgccac acccagccgg 3120

ccacagtcga tgaatccaga aaagcggcca ttttccacca tgatattcgg caagcaggca 3180

tcgccatggg tcacgacgag atcctcgccg tcgggcatgc tcgccttgag cctggcgaac 3240

agttcggctg gcgcgagccc ctgatgctct tgatcatcct gatcgacaag accggcttcc 3300

atccgagtac gtgctcgctc gatgcgatgt ttcgcttggt ggtcgaatgg gcaggtagcc 3360

ggatcaagcg tatgcagccg ccgcattgca tcagccatga tggatacttt ctcggcagga 3420

gcaaggtgag atgacaggag atcctgcccc ggcacttcgc ccaatagcag ccagtccctt 3480

cccgcttcag tgacaacgtc gagcacagct gcgcaaggaa cgcccgtcgt ggccagccac 3540

gatagccgcg ctgcctcgtc ttgcagttca ttcagggcac cggacaggtc ggtcttgaca 3600

aaaagaaccg ggcgcccctg cgctgacagc cggaacacgg cggcatcaga gcagccgatt 3660

gtctgttgtg cccagtcata gccgaatagc ctctccaccc aagcggccgg agaacctgcg 3720

tgcaatccat cttgttcaat catgcgaaac gatcctcatc ctgtctcttg atcgatcttt 3780

gcaaaagcct aggcctccaa aaaagcctcc tcactacttc tggaatagct cagaggccga 3840

ggcggcctcg gcctctgcat aaataaaaaa aattagtcag ccatggggcg gagaatgggc 3900

ggaactgggc ggagttaggg gcgggatggg cggagttagg ggcgggacta tggttgctga 3960

ctaattgaga tgcatgcttt gcatacttct gcctgctggg gagcctgggg actttccaca 4020

cctggttgct gactaattga gatgcatgct ttgcatactt ctgcctgctg gggagcctgg 4080

ggactttcca caccctaact gacacacatt ccacagctgg ttctttccgc ctcaggactc 4140

ttcctttttc aataaatcaa tctaaagtat atatgagtaa acttggtctg acagttacca 4200

atgcttaatc agtgaggcac ctatctcagc gatctgtcta tttcgttcat ccatagttgc 4260

ctgactcccc gtcgtgtaga taactacgat acgggagggc ttaccatctg gccccagtgc 4320

tgcaatgata ccgcgagacc cacgctcacc ggctccagat ttatcagcaa taaaccagcc 4380

agccggaagg gccgagcgca gaagtggtcc tgcaacttta tccgcctcca tccagtctat 4440

taattgttgc cgggaagcta gagtaagtag ttcgccagtt aatagtttgc gcaacgttgt 4500

tgccattgct acaggcatcg tggtgtcacg ctcgtcgttt ggtatggctt cattcagctc 4560

cggttcccaa cgatcaaggc gagttacatg atcccccatg ttgtgcaaaa aagcggttag 4620

ctccttcggt cctccgatcg ttgtcagaag taagttggcc gcagtgttat cactcatggt 4680

tatggcagca ctgcataatt ctcttactgt catgccatcc gtaagatgct tttctgtgac 4740

tggtgagtac tcaaccaagt cattctgaga atagtgtatg cggcgaccga gttgctcttg 4800

cccggcgtca atacgggata ataccgcgcc acatagcaga actttaaaag tgctcatcat 4860

tggaaaacgt tcttcggggc gaaaactctc aaggatctta ccgctgttga gatccagttc 4920

gatgtaaccc actcgtgcac ccaactgatc ttcagcatct tttactttca ccagcgtttc 4980

tgggtgagca aaaacaggaa ggcaaaatgc cgcaaaaaag ggaataaggg cgacacggaa 5040

atgttgaata ctcatactct tcctttttca atattattga agcatttatc agggttattg 5100

tctcatgagc ggatacatat ttgaatgtat ttagaaaaat aaacaaatag gggttccgcg 5160

cacatttccc cgaaaagtgc cacctgacgc gccctgtagc ggcgcattaa gcgcggcggg 5220

tgtggtggtt acgcgcagcg tgaccgctac acttgccagc gccctagcgc ccgctccttt 5280

cgctttcttc ccttcctttc tcgccacgtt cgccggcttt ccccgtcaag ctctaaatcg 5340

ggggctccct ttagggttcc gatttagtgc tttacggcac ctcgacccca aaaaacttga 5400

ttagggtgat ggttcacgta gtgggccatc gccctgatag acggtttttc gccctttgac 5460

gttggagtcc acgttcttta atagtggact cttgttccaa actggaacaa cactcaaccc 5520

tatctcggtc tattcttttg atttataagg gattttgccg atttcggcct attggttaaa 5580

aaatgagctg atttaacaaa aatttaacgc gaattttaac aaaatattaa cgct 5634

<210> 8
<211> 5606
<212> DNA
<213> artificial

<220>
<223> Plasmid VR_GP5; BamH I / Xho I; Cloning of GP5 PRRSV VR-2332 into
pCR3.1

<220>
<221> misc_feature
<222> (682)..(1304)
<223> BamH I - Xho I ; PRRSV VR-2332 GP5

<400> 8
gcgcgcgttg acattgatta ttgactagtt attaatagta atcaattacg gggtcattag 60

ttcatagccc atatatggag ttccgcgtta cataacttac ggtaaatggc ccgcctggct 120

gaccgcccaa cgacccccgc ccattgacgt caataatgac gtatgttccc atagtaacgc 180

caatagggac tttccattga cgtcaatggg tggactattt acggtaaact gcccacttgg 240

cagtacatca agtgtatcat atgccaagta cgccccctat tgacgtcaat gacggtaaat 300

ggcccgcctg gcattatgcc cagtacatga ccttatggga ctttcctact tggcagtaca 360

tctacgtatt agtcatcgct attaccatgg tgatgcggtt ttggcagtac atcaatgggc 420

gtggatagcg gtttgactca cggggatttc caagtctcca ccccattgac gtcaatggga 480

gtttgttttg gcaccaaaat caacgggact ttccaaaatg tcgtaacaac tccgccccat 540

tgacgcaaat gggcggtagg cgtgtacggt gggaggtcta tataagcaga gctctctggc 600

taactagaga acccactgct tactggctta tcgaaattaa tacgactcac tatagggaga 660

cccaagcttg gtaccgagct cggatccgcc atgttggaga aatgcttgac cgcgggctgt 720

tgctcgcgat tgctttcttt gtggtgtatc gtgccgttct gttttgctgt gctcgccaac 780

gccagcaacg acagcagctc ccatctacag ctgatttaca acttgacgct atgtgagctg 840

aatggcacag attggctagc taacaaattt gattgggcag tggagagttt tgtcatcttt 900

cccgttttga ctcacattgt ctcctatggt gccctcacta ccagccattt ccttgacaca 960

gtcgctttag tcactgtgtc taccgccggg tttgttcacg ggcggtatgt cctaagtagc 1020

atctacgcgg tctgtgccct ggctgcgttg acttgcttcg tcattaggtt tgcaaagaat 1080

tgcatgtcct ggcgctacgc gtgtaccaga tataccaact ttcttctgga cactaagggc 1140

agactctatc gttggcggtc gcctgtcatc atagagaaaa ggggcaaagt tgaggtcgaa 1200

ggtcatctga tcgacctcaa aagagttgtg cttgatggtt ccgtggcaac ccctataacc 1260

agagtttcag cggaacaatg gggtcgtcct tagatgactt ctgctcgagc atgcatctag 1320

agggccctat tctatagtgt cacctaaatg ctagagctcg ctgatcagcc tcgactgtgc 1380

cttctagttg ccagccatct gttgtttgcc cctcccccgt gccttccttg accctggaag 1440

gtgccactcc cactgtcctt tcctaataaa atgaggaaat tgcatcgcat tgtctgagta 1500

ggtgtcattc tattctgggg ggtggggtgg ggcaggacag caagggggag gattgggaag 1560

acaatagcag gcatgctggg gatgcggtgg gctctatggc ttctgaggcg gaaagaacca 1620

gtggcggtaa tacggttatc cacagaatca ggggataacg caggaaagaa catgtgagca 1680

aaaggccagc aaaaggccag gaaccgtaaa aaggccgcgt tgctggcgtt tttccatagg 1740

ctccgccccc ctgacgagca tcacaaaaat cgacgctcaa gtcagaggtg gcgaaacccg 1800

acaggactat aaagatacca ggcgtttccc cctggaagct ccctcgtgcg ctctcctgtt 1860

ccgaccctgc cgcttaccgg atacctgtcc gcctttctcc cttcgggaag cgtggcgctt 1920

tctcatagct cacgctgtag gtatctcagt tcggtgtagg tcgttcgctc caagctgggc 1980

tgtgtgcacg aaccccccgt tcagcccgac cgctgcgcct tatccggtaa ctatcgtctt 2040

gagtccaacc cggtaagaca cgacttatcg ccactggcag cagccactgg taacaggatt 2100

agcagagcga ggtatgtagg cggtgctaca gagttcttga agtggtggcc taactacggc 2160

tacactagaa ggacagtatt tggtatctgc gctctgctga agccagttac cttcggaaaa 2220

agagttggta gctcttgatc cggcaaacaa accaccgctg gtagcggtgg tttttttgtt 2280

tgcaagcagc agattacgcg cagaaaaaaa ggatctcaag aagatccttt gatcttttct 2340

acggggtctg acgctcagtg gaacgaaaac tcacgttaag ggattttggt catgagatta 2400

tcaaaaagga tcttcaccta gatcctttta aattaaaaat gaagttttaa atcaatctaa 2460

agtatatatg agtaacctga ggctatggca gggcctgccg ccccgacgtt ggctgcgagc 2520

cctgggcctt cacccgaact tgggggtggg gtggggaaaa ggaagaaacg cgggcgtatt 2580

tggccccaat ggggtctcgg tggggtatcg acagagtgcc agccctggga ccgaaccccg 2640

cgtttatgaa caaacgaccc aacaccgtgc gttttattct gtctttttat tgccgtcata 2700

gcgcgggttc cttccggtat tgtctccttc cgtgtttcag ttagcctccc cctagggtgg 2760

gcgaagaact ccagcatgag atccccgcgc tggaggatca tccagccggc gtcccggaaa 2820

acgattccga agcccaacct ttcatagaag gcggcggtgg aatcgaaatc tcgtgatggc 2880

aggttgggcg tcgcttggtc ggtcatttcg aaccccagag tcccgctcag aagaactcgt 2940

caagaaggcg atagaaggcg atgcgctgcg aatcgggagc ggcgataccg taaagcacga 3000

ggaagcggtc agcccattcg ccgccaagct cttcagcaat atcacgggta gccaacgcta 3060

tgtcctgata gcggtccgcc acacccagcc ggccacagtc gatgaatcca gaaaagcggc 3120

cattttccac catgatattc ggcaagcagg catcgccatg ggtcacgacg agatcctcgc 3180

cgtcgggcat gctcgccttg agcctggcga acagttcggc tggcgcgagc ccctgatgct 3240

cttgatcatc ctgatcgaca agaccggctt ccatccgagt acgtgctcgc tcgatgcgat 3300

gtttcgcttg gtggtcgaat gggcaggtag ccggatcaag cgtatgcagc cgccgcattg 3360

catcagccat gatggatact ttctcggcag gagcaaggtg agatgacagg agatcctgcc 3420

ccggcacttc gcccaatagc agccagtccc ttcccgcttc agtgacaacg tcgagcacag 3480

ctgcgcaagg aacgcccgtc gtggccagcc acgatagccg cgctgcctcg tcttgcagtt 3540

cattcagggc accggacagg tcggtcttga caaaaagaac cgggcgcccc tgcgctgaca 3600

gccggaacac ggcggcatca gagcagccga ttgtctgttg tgcccagtca tagccgaata 3660

gcctctccac ccaagcggcc ggagaacctg cgtgcaatcc atcttgttca atcatgcgaa 3720

acgatcctca tcctgtctct tgatcgatct ttgcaaaagc ctaggcctcc aaaaaagcct 3780

cctcactact tctggaatag ctcagaggcc gaggcggcct cggcctctgc ataaataaaa 3840

aaaattagtc agccatgggg cggagaatgg gcggaactgg gcggagttag gggcgggatg 3900

ggcggagtta ggggcgggac tatggttgct gactaattga gatgcatgct ttgcatactt 3960

ctgcctgctg gggagcctgg ggactttcca cacctggttg ctgactaatt gagatgcatg 4020

ctttgcatac ttctgcctgc tggggagcct ggggactttc cacaccctaa ctgacacaca 4080

ttccacagct ggttctttcc gcctcaggac tcttcctttt tcaataaatc aatctaaagt 4140

atatatgagt aaacttggtc tgacagttac caatgcttaa tcagtgaggc acctatctca 4200

gcgatctgtc tatttcgttc atccatagtt gcctgactcc ccgtcgtgta gataactacg 4260

atacgggagg gcttaccatc tggccccagt gctgcaatga taccgcgaga cccacgctca 4320

ccggctccag atttatcagc aataaaccag ccagccggaa gggccgagcg cagaagtggt 4380

cctgcaactt tatccgcctc catccagtct attaattgtt gccgggaagc tagagtaagt 4440

agttcgccag ttaatagttt gcgcaacgtt gttgccattg ctacaggcat cgtggtgtca 4500

cgctcgtcgt ttggtatggc ttcattcagc tccggttccc aacgatcaag gcgagttaca 4560

tgatccccca tgttgtgcaa aaaagcggtt agctccttcg gtcctccgat cgttgtcaga 4620

agtaagttgg ccgcagtgtt atcactcatg gttatggcag cactgcataa ttctcttact 4680

gtcatgccat ccgtaagatg cttttctgtg actggtgagt actcaaccaa gtcattctga 4740

gaatagtgta tgcggcgacc gagttgctct tgcccggcgt caatacggga taataccgcg 4800

ccacatagca gaactttaaa agtgctcatc attggaaaac gttcttcggg gcgaaaactc 4860

tcaaggatct taccgctgtt gagatccagt tcgatgtaac ccactcgtgc acccaactga 4920

tcttcagcat cttttacttt caccagcgtt tctgggtgag caaaaacagg aaggcaaaat 4980

gccgcaaaaa agggaataag ggcgacacgg aaatgttgaa tactcatact cttccttttt 5040

caatattatt gaagcattta tcagggttat tgtctcatga gcggatacat atttgaatgt 5100

atttagaaaa ataaacaaat aggggttccg cgcacatttc cccgaaaagt gccacctgac 5160

gcgccctgta gcggcgcatt aagcgcggcg ggtgtggtgg ttacgcgcag cgtgaccgct 5220

acacttgcca gcgccctagc gcccgctcct ttcgctttct tcccttcctt tctcgccacg 5280

ttcgccggct ttccccgtca agctctaaat cgggggctcc ctttagggtt ccgatttagt 5340

gctttacggc acctcgaccc caaaaaactt gattagggtg atggttcacg tagtgggcca 5400

tcgccctgat agacggtttt tcgccctttg acgttggagt ccacgttctt taatagtgga 5460

ctcttgttcc aaactggaac aacactcaac cctatctcgg tctattcttt tgatttataa 5520

gggattttgc cgatttcggc ctattggtta aaaaatgagc tgatttaaca aaaatttaac 5580

gcgaatttta acaaaatatt aacgct 5606

<210> 9
<211> 5629
<212> DNA
<213> artificial

<220>
<223> Plasmid GP5_HA; BamH I / Xho I; Cloning of GP5 PRRSV Lelystad +
      HA Tag into pCR3.1


<220>
<221> misc_feature
<222> (682)..(1327)
<223> BamH I - Xho I ; PRRSV lelystad GP5 + HA-tag

<400> 9
gcgcgcgttg acattgatta ttgactagtt attaatagta atcaattacg gggtcattag      60

ttcatagccc atatatggag ttccgcgtta cataacttac ggtaaatggc ccgcctggct     120

gaccgcccaa cgacccccgc ccattgacgt caataatgac gtatgttccc atagtaacgc     180

caatagggac tttccattga cgtcaatggg tggactattt acggtaaact gcccacttgg     240

cagtacatca agtgtatcat atgccaagta cgccccctat tgacgtcaat gacggtaaat     300

ggcccgcctg gcattatgcc cagtacatga ccttatggga ctttcctact tggcagtaca     360

tctacgtatt agtcatcgct attaccatgg tgatgcggtt ttggcagtac atcaatgggc     420

gtggatagcg gtttgactca cggggatttc caagtctcca ccccattgac gtcaatggga     480

gtttgttttg gcaccaaaat caacgggact ttccaaaatg tcgtaacaac tccgccccat     540

tgacgcaaat gggcggtagg cgtgtacggt gggaggtcta tataagcaga gctctctggc     600

taactagaga acccactgct tactggctta tcgaaattaa tacgactcac tatagggaga     660

cccaagcttg gtaccgagct cggatccacc atgagatgtt ctcacaaatt ggggcgtttc     720

ttgactccgc actcttgctt ctggtggttt tttttgctgt gtaccggctt gtcctggtcc     780

tttgccgatg gcaacggcaa cagctcgaca taccaataca tatataactt gacgatatgc     840

gagctgaatg ggaccgactg gttgtccagc cattttggtt gggcagtcga gacctttgtg     900

ttttacccgg ttgccactca tatcctctca ctgggttttc tcacaacaag ccattttttt     960

gacgcgctcg gtctcggcgc tgtatccact gcaggatttg ttggcgggcg gtatgtactc    1020

tgcagcgtct acggcgcttg tgctttcgca gcgttcgtat gttttgtcat ccgtgctgct    1080

aaaaattgca tggcctgccg ctatgcccgt acccggttta ccaacttcat tgtagacaac    1140

cgggggagag ttcatcgatg gaagtctcca atagtggtag aaaaattggg caaagccgaa    1200

gtcgacggca acctcgtcac catcaaacat gtcgtcctcg aaggggttaa agctcaaccc    1260

ttgacgagga cttcggctga gcaatgggag gccgcatacc cctacgatgt acccgattac    1320

gcatgactcg agcatgcatc tagagggccc tattctatag tgtcacctaa atgctagagc    1380

tcgctgatca gcctcgactg tgccttctag ttgccagcca tctgttgttt gcccctcccc 1440
cgtgccttcc ttgaccctgg aaggtgccac tcccactgtc ctttcctaat aaaatgagga 1500
aattgcatcg cattgtctga gtaggtgtca ttctattctg gggggtgggg tggggcagga 1560
cagcaagggg gaggattggg aagacaatag caggcatgct ggggatgcgg tgggctctat 1620
ggcttctgag gcggaaagaa ccagtggcgg taatacggtt atccacagaa tcaggggata 1680
acgcaggaaa gaacatgtga gcaaaaggcc agcaaaaggc caggaaccgt aaaaaggccg 1740
cgttgctggc gtttttccat aggctccgcc cccctgacga gcatcacaaa aatcgacgct 1800
caagtcagag gtggcgaaac ccgacaggac tataaagata ccaggcgttt ccccctggaa 1860
gctccctcgt gcgctctcct gttccgaccc tgccgcttac cggatacctg tccgcctttc 1920
tcccttcggg aagcgtggcg ctttctcata gctcacgctg taggtatctc agttcggtgt 1980
aggtcgttcg ctccaagctg ggctgtgtgc acgaaccccc cgttcagccc gaccgctgcg 2040
ccttatccgg taactatcgt cttgagtcca acccggtaag acacgactta tcgccactgg 2100
cagcagccac tggtaacagg attagcagag cgaggtatgt aggcggtgct acagagttct 2160
tgaagtggtg gcctaactac ggctacacta gaaggacagt atttggtatc tgcgctctgc 2220
tgaagccagt taccttcgga aaaagagttg gtagctcttg atccggcaaa caaaccaccg 2280
ctggtagcgg tggttttttt gtttgcaagc agcagattac gcgcagaaaa aaaggatctc 2340
aagaagatcc tttgatcttt tctacggggt ctgacgctca gtggaacgaa aactcacgtt 2400
aagggatttt ggtcatgaga ttatcaaaaa ggatcttcac ctagatcctt ttaaattaaa 2460
aatgaagttt taaatcaatc taaagtatat atgagtaacc tgaggctatg gcagggcctg 2520
ccgccccgac gttggctgcg agccctgggc cttcacccga acttgggggg tggggtgggg 2580
aaaaggaaga aacgcgggcg tattggcccc aatggggtct cggtggggta tcgacagagt 2640
gccagccctg ggaccgaacc ccgcgtttat gaacaaacga cccaacaccg tgcgttttat 2700
tctgtctttt tattgccgtc atagcgcggg ttccttccgg tattgtctcc ttccgtgttt 2760
cagttagcct ccccctaggg tgggcgaaga actccagcat gagatccccg cgctggagga 2820
tcatccagcc ggcgtcccgg aaaacgattc cgaagcccaa cctttcatag aaggcggcgg 2880
tggaatcgaa atctcgtgat ggcaggttgg gcgtcgcttg gtcggtcatt tcgaacccca 2940
gagtcccgct cagaagaact cgtcaagaag gcgatagaag gcgatgcgct gcgaatcggg 3000
agcggcgata ccgtaaagca cgaggaagcg gtcagcccat tcgccgccaa gctcttcagc 3060
aatatcacgg gtagccaacg ctatgtcctg atagcggtcc gccacaccca gccggccaca 3120
gtcgatgaat ccagaaaagc ggccattttc caccatgata ttcggcaagc aggcatcgcc 3180
atgggtcacg acgagatcct cgccgtcggg catgctcgcc ttgagcctgg cgaacagttc 3240

ggctggcgcg agcccctgat gctcttgatc atcctgatcg acaagaccgg cttccatccg 3300

agtacgtgct cgctcgatgc gatgtttcgc ttggtggtcg aatgggcagg tagccggatc 3360

aagcgtatgc agccgccgca ttgcatcagc catgatggat actttctcgg caggagcaag 3420

gtgagatgac aggagatcct gccccggcac ttcgcccaat agcagccagt cccttcccgc 3480

ttcagtgaca acgtcgagca cagctgcgca aggaacgccc gtcgtggcca gccacgatag 3540

ccgcgctgcc tcgtcttgca gttcattcag ggcaccggac aggtcggtct tgacaaaaag 3600

aaccgggcgc ccctgcgctg acagccggaa cacggcggca tcagagcagc cgattgtctg 3660

ttgtgcccag tcatagccga atagcctctc cacccaagcg gccggagaac ctgcgtgcaa 3720

tccatcttgt tcaatcatgc gaaacgatcc tcatcctgtc tcttgatcga tctttgcaaa 3780

agcctaggcc tccaaaaaag cctcctcact acttctggaa tagctcagag gccgaggcgg 3840

cctcggcctc tgcataaata aaaaaaatta gtcagccatg gggcggagaa tgggcggaac 3900

tgggcggagt taggggcggg atgggcggag ttaggggcgg gactatggtt gctgactaat 3960

tgagatgcat gctttgcata cttctgcctg ctggggagcc tggggacttt ccacacctgg 4020

ttgctgacta attgagatgc atgctttgca tacttctgcc tgctggggag cctggggact 4080

ttccacaccc taactgacac acattccaca gctggttctt tccgcctcag gactcttcct 4140

ttttcaataa atcaatctaa agtatatatg agtaaacttg gtctgacagt taccaatgct 4200

taatcagtga ggcacctatc tcagcgatct gtctatttcg ttcatccata gttgcctgac 4260

tccccgtcgt gtagataact acgatacggg agggcttacc atctggcccc agtgctgcaa 4320

tgataccgcg agacccacgc tcaccggctc cagatttatc agcaataaac cagccagccg 4380

gaagggccga gcgcagaagt ggtcctgcaa ctttatccgc ctccatccag tctattaatt 4440

gttgccggga agctagagta agtagttcgc cagttaatag tttgcgcaac gttgttgcca 4500

ttgctacagg catcgtggtg tcacgctcgt cgtttggtat ggcttcattc agctccggtt 4560

cccaacgatc aaggcgagtt acatgatccc ccatgttgtg caaaaaagcg gttagctcct 4620

tcggtcctcc gatcgttgtc agaagtaagt tggccgcagt gttatcactc atggttatgg 4680

cagcactgca taattctctt actgtcatgc catccgtaag atgcttttct gtgactggtg 4740

agtactcaac caagtcattc tgagaatagt gtatgcggcg accgagttgc tcttgcccgg 4800

cgtcaatacg ggataatacc gcgccacata gcagaacttt aaaagtgctc atcattggaa 4860

aacgttcttc ggggcgaaaa ctctcaagga tcttaccgct gttgagatcc agttcgatgt 4920

aacccactcg tgcacccaac tgatcttcag catcttttac tttcaccagc gtttctgggt 4980

gagcaaaaac aggaaggcaa aatgccgcaa aaaagggaat aagggcgaca cggaaatgtt 5040

gaatactcat actcttcctt tttcaatatt attgaagcat ttatcagggt tattgtctca 5100

tgagcggata catatttgaa tgtatttaga aaaataaaca aataggggtt ccgcgcacat 5160

ttccccgaaa agtgccacct gacgcgccct gtagcggcgc attaagcgcg gcgggtgtgg 5220

tggttacgcg cagcgtgacc gctacacttg ccagcgccct agcgcccgct cctttcgctt 5280

tcttcccttc ctttctcgcc acgttcgccg gctttccccg tcaagctcta aatcgggggc 5340

tccctttagg gttccgattt agtgctttac ggcacctcga ccccaaaaaa cttgattagg 5400

gtgatggttc acgtagtggg ccatcgccct gatagacggt ttttcgccct ttgacgttgg 5460

agtccacgtt ctttaatagt ggactcttgt tccaaactgg aacaacactc aaccctatct 5520

cggtctattc ttttgattta taagggattt tgccgatttc ggcctattgg ttaaaaaatg 5580

agctgattta acaaaaattt aacgcgaatt ttaacaaaat attaacgct 5629

<210> 10
<211> 5626
<212> DNA
<213> artificial

<220>
<223> Plasmid VR_GP5; BamH I / Xho I; Cloning of GP5 PRRSV VR-2332 +
HA-tag into
pCR3.1

<220>
<221> misc_feature
<222> (682)..(1324)
<223> BamH I - Xho I ; PRRSV VR-2332 GP5

<400> 10
gcgcgcgttg acattgatta ttgactagtt attaatagta atcaattacg gggtcattag 60

ttcatagccc atatatggag ttccgcgtta cataacttac ggtaaatggc ccgcctggct 120

gaccgcccaa cgacccccgc ccattgacgt caataatgac gtatgttccc atagtaacgc 180

caatagggac tttccattga cgtcaatggg tggactattt acggtaaact gcccacttgg 240

cagtacatca agtgtatcat atgccaagta cgccccctat tgacgtcaat gacggtaaat 300

ggcccgcctg gcattatgcc cagtacatga ccttatggga ctttcctact tggcagtaca 360

tctacgtatt agtcatcgct attaccatgg tgatgcggtt ttggcagtac atcaatgggc 420

gtggatagcg gtttgactca cggggatttc caagtctcca ccccattgac gtcaatggga 480

gtttgttttg gcaccaaaat caacgggact ttccaaaatg tcgtaacaac tccgccccat 540

tgacgcaaat gggcggtagg cgtgtacggt gggaggtcta tataagcaga gctctctggc 600

taactagaga acccactgct tactggctta tcgaaattaa tacgactcac tatagggaga 660

cccaagcttg gtaccgagct cggatccgcc atgttggaga aatgcttgac cgcgggctgt 720

tgctcgcgat tgctttcttt gtggtgtatc gtgccgttct gttttgctgt gctcgccaac 780

gccagcaacg acagcagctc ccatctacag ctgatttaca acttgacgct atgtgagctg 840

aatggcacag attggctagc taacaaattt gattgggcag tggagagttt tgtcatcttt 900

cccgttttga ctcacattgt ctcctatggt gccctcacta ccagccattt ccttgacaca 960

gtcgctttag tcactgtgtc taccgccggg tttgttcacg ggcggtatgt cctaagtagc 1020

atctacgcgg tctgtgccct ggctgcgttg acttgcttcg tcattaggtt tgcaaagaat 1080

tgcatgtcct ggcgctacgc gtgtaccaga tataccaact ttcttctgga cactaagggc 1140

agactctatc gttggcggtc gcctgtcatc atagagaaaa ggggcaaagt tgaggtcgaa 1200

ggtcatctga tcgacctcaa aagagttgtg cttgatggtt ccgtggcaac ccctataacc 1260

agagtttcag cggaacaatg gggtcgtcct gcatacccct acgatgtacc cgattacgca 1320

tgactcgagc atgcatctag agggccctat tctatagtgt cacctaaatg ctagagctcg 1380

ctgatcagcc tcgactgtgc cttctagttg ccagccatct gttgtttgcc cctcccccgt 1440

gccttccttg accctggaag gtgccactcc cactgtcctt tcctaataaa atgaggaaat 1500

tgcatcgcat tgtctgagta ggtgtcattc tattctgggg ggtggggtgg ggcaggacag 1560

caagggggag gattgggaag acaatagcag gcatgctggg gatgcggtgg gctctatggc 1620

ttctgaggcg gaaagaacca gtggcggtaa tacggttatc cacagaatca ggggataacg 1680

caggaaagaa catgtgagca aaaggccagc aaaaggccag gaaccgtaaa aaggccgcgt 1740

tgctggcgtt tttccatagg ctccgccccc ctgacgagca tcacaaaaat cgacgctcaa 1800

gtcagaggtg gcgaaacccg acaggactat aaagatacca ggcgtttccc cctggaagct 1860

ccctcgtgcg ctctcctgtt ccgaccctgc cgcttaccgg atacctgtcc gcctttctcc 1920

cttcgggaag cgtggcgctt tctcatagct cacgctgtag gtatctcagt tcggtgtagg 1980

tcgttcgctc caagctgggc tgtgtgcacg aaccccccgt tcagcccgac cgctgcgcct 2040

tatccggtaa ctatcgtctt gagtccaacc cggtaagaca cgacttatcg ccactggcag 2100

cagccactgg taacaggatt agcagagcga ggtatgtagg cggtgctaca gagttcttga 2160

agtggtggcc taactacggc tacactagaa ggacagtatt tggtatctgc gctctgctga 2220

agccagttac cttcggaaaa agagttggta gctcttgatc cggcaaacaa accaccgctg 2280

gtagcggtgg tttttttgtt tgcaagcagc agattacgcg cagaaaaaaa ggatctcaag 2340

aagatccttt gatcttttct acggggtctg acgctcagtg gaacgaaaac tcacgttaag 2400

ggattttggt catgagatta tcaaaaagga tcttcaccta gatcctttta aattaaaaat 2460

gaagttttaa atcaatctaa agtatatatg agtaacctga ggctatggca gggcctgccg 2520

ccccgacgtt ggctgcgagc cctgggcctt cacccgaact tggggggtgg ggtggggaaa 2580

aggaagaaac gcgggcgtat tggccccaat ggggtctcgg tggggtatcg acagagtgcc 2640

agccctggga ccgaaccccg cgtttatgaa caaacgaccc aacaccgtgc gttttattct 2700

gtctttttat tgccgtcata gcgcgggttc cttccggtat tgtctccttc cgtgtttcag 2760

ttagcctccc cctagggtgg gcgaagaact ccagcatgag atccccgcgc tggaggatca 2820
tccagccggc gtcccggaaa acgattccga agcccaacct ttcatagaag gcggcggtgg 2880
aatcgaaatc tcgtgatggc aggttgggcg tcgcttggtc ggtcatttcg aaccccagag 2940
tcccgctcag aagaactcgt caagaaggcg atagaaggcg atgcgctgcg aatcgggagc 3000
ggcgataccg taaagcacga ggaagcggtc agcccattcg ccgccaagct cttcagcaat 3060
atcacgggta gccaacgcta tgtcctgata gcggtccgcc acacccagcc ggccacagtc 3120
gatgaatcca gaaaagcggc cattttccac catgatattc ggcaagcagg catcgccatg 3180
ggtcacgacg agatcctcgc cgtcgggcat gctcgccttg agcctggcga acagttcggc 3240
tggcgcgagc ccctgatgct cttgatcatc ctgatcgaca agaccggctt ccatccgagt 3300
acgtgctcgc tcgatgcgat gtttcgcttg gtggtcgaat gggcaggtag ccggatcaag 3360
cgtatgcagc cgccgcattg catcagccat gatggatact ttctcggcag gagcaaggtg 3420
agatgacagg agatcctgcc ccggcacttc gcccaatagc agccagtccc ttcccgcttc 3480
agtgacaacg tcgagcacag ctgcgcaagg aacgcccgtc gtggccagcc acgatagccg 3540
cgctgcctcg tcttgcagtt cattcagggc accggacagg tcggtcttga caaaaagaac 3600
cgggcgcccc tgcgctgaca gccggaacac ggcggcatca gagcagccga ttgtctgttg 3660
tgcccagtca tagccgaata gcctctccac ccaagcggcc ggagaacctg cgtgcaatcc 3720
atcttgttca atcatgcgaa acgatcctca tcctgtctct tgatcgatct ttgcaaaagc 3780
ctaggcctcc aaaaaagcct cctcactact tctggaatag ctcagaggcc gaggcggcct 3840
cggcctctgc ataaataaaa aaaattagtc agccatgggg cggagaatgg gcggaactgg 3900
gcggagttag gggcgggatg ggcggagtta ggggcgggac tatggttgct gactaattga 3960
gatgcatgct ttgcatactt ctgcctgctg gggagcctgg ggactttcca cacctggttg 4020
ctgactaatt gagatgcatg ctttgcatac ttctgcctgc tggggagcct ggggactttc 4080
cacaccctaa ctgacacaca ttccacagct ggttctttcc gcctcaggac tcttcctttt 4140
tcaataaatc aatctaaagt atatatgagt aaacttggtc tgacagttac caatgcttaa 4200
tcagtgaggc acctatctca gcgatctgtc tatttcgttc atccatagtt gcctgactcc 4260
ccgtcgtgta gataactacg atacgggagg gcttaccatc tggccccagt gctgcaatga 4320
taccgcgaga cccacgctca ccggctccag atttatcagc aataaaccag ccagccggaa 4380
gggccgagcg cagaagtggt cctgcaactt tatccgcctc catccagtct attaattgtt 4440
gccgggaagc tagagtaagt agttcgccag ttaatagttt gcgcaacgtt gttgccattg 4500
ctacaggcat cgtggtgtca cgctcgtcgt ttggtatggc ttcattcagc tccggttccc 4560
aacgatcaag gcgagttaca tgatccccca tgttgtgcaa aaaagcggtt agctccttcg 4620

gtcctccgat cgttgtcaga agtaagttgg ccgcagtgtt atcactcatg gttatggcag 4680

cactgcataa ttctcttact gtcatgccat ccgtaagatg cttttctgtg actggtgagt 4740

actcaaccaa gtcattctga gaatagtgta tgcggcgacc gagttgctct tgcccggcgt 4800

caatacggga taataccgcg ccacatagca gaactttaaa agtgctcatc attggaaaac 4860

gttcttcggg gcgaaaactc tcaaggatct taccgctgtt gagatccagt tcgatgtaac 4920

ccactcgtgc acccaactga tcttcagcat cttttacttt caccagcgtt tctgggtgag 4980

caaaaacagg aaggcaaaat gccgcaaaaa agggaataag ggcgacacgg aaatgttgaa 5040

tactcatact cttccttttt caatattatt gaagcattta tcagggttat tgtctcatga 5100

gcggatacat atttgaatgt atttagaaaa ataaacaaat aggggttccg cgcacatttc 5160

cccgaaaagt gccacctgac gcgccctgta gcggcgcatt aagcgcggcg ggtgtggtgg 5220

ttacgcgcag cgtgaccgct acacttgcca gcgccctagc gcccgctcct ttcgctttct 5280

tcccttcctt tctcgccacg ttcgccggct ttccccgtca agctctaaat cgggggctcc 5340

ctttagggtt ccgatttagt gctttacggc acctcgaccc caaaaaactt gattagggtg 5400

atggttcacg tagtgggcca tcgccctgat agacggtttt tcgccctttg acgttggagt 5460

ccacgttctt taatagtgga ctcttgttcc aaactggaac aacactcaac cctatctcgg 5520

tctattcttt tgatttataa gggattttgc cgatttcggc ctattggtta aaaaatgagc 5580

tgatttaaca aaaatttaac gcgaatttta acaaaatatt aacgct 5626

<210> 11
<211> 5776
<212> DNA
<213> Artificial

<220>
<223> Plasmid EAV_GP5; BamH I / Xho I; Cloning of GP5 EAV into pCR3.1

<220>
<221> misc_feature
<222> (682)..(1474)
<223> BamH I - Xho I ; EAV GP5

<400> 11
gcgcgcgttg acattgatta ttgactagtt attaatagta atcaattacg gggtcattag 60

ttcatagccc atatatggag ttccgcgtta cataacttac ggtaaatggc ccgcctggct 120

gaccgcccaa cgacccccgc ccattgacgt caataatgac gtatgttccc atagtaacgc 180

caatagggac tttccattga cgtcaatggg tggactattt acggtaaact gcccacttgg 240

cagtacatca agtgtatcat atgccaagta cgccccctat tgacgtcaat gacggtaaat 300

ggcccgcctg gcattatgcc cagtacatga ccttatggga ctttcctact tggcagtaca 360

tctacgtatt agtcatcgct attaccatgg tgatgcggtt ttggcagtac atcaatgggc 420

gtggatagcg gtttgactca cggggatttc caagtctcca ccccattgac gtcaatggga 480

gtttgttttg gcaccaaaat caacgggact ttccaaaatg tcgtaacaac tccgccccat 540

tgacgcaaat gggcggtagg cgtgtacggt gggaggtcta tataagcaga gctctctggc 600

taactagaga acccactgct tactggctta tcgaaattaa tacgactcac tatagggaga 660

cccaagcttg gtaccgagct cggatccggt acgttgggct caacaatgtt atctatgatt 720

gtactgctat tcttgctttg gggtgcgcca tcacatgctt acttctcata ctacaccgct 780

cagcgcttca cagacttcac cttgtgtatg ctgacggatc gcggcgttat tgccaatttg 840

ctgcgatatg atgagcacac tgctttgtac aattgttccg ccagtaaaac ctgttggtat 900

tgcacattcc tggacgaaca gattatcacg tttggaaccg attgtaatga cacctacgcg 960

gtcccagttg ctgaggtcct ggaacaggcg catggaccgt acagtgtgct gtttaatgac 1020

atgccccctt ttatttacta tggccgtgaa ttcggcatag ttgtgttgga tgtgtttatg 1080

ttctatcccg ttttagttct gtttttctta tcagtactac cctatgctac gcttattctt 1140

gaaatgtgtg tatctattct gtttataatc tatggcattt acagcggggc ctacttggcc 1200

atgggcatat ttgcggccac gcttgctata cattcaattg tggtcctccg ccaattactg 1260

tggttatgcc tggcttggcg ataccgctgt acgcttcacg cgtcctttat atcagctgag 1320

gggaaagtgt accccgtaga ccccggactc ccggttgccg ccgcgggcaa tcggttgtta 1380

gtcccaggta ggcccactat cgattatgca gtggcctacg gcagcaaagt caaccttgtg 1440

aggttggggg cagctgaggt atgggagcca tagctcgagc atgcatctag agggccctat 1500

tctatagtgt cacctaaatg ctagagctcg ctgatcagcc tcgactgtgc cttctagttg 1560

ccagccatct gttgtttgcc cctcccccgt gccttccttg accctggaag gtgccactcc 1620

cactgtcctt tcctaataaa atgaggaaat tgcatcgcat tgtctgagta ggtgtcattc 1680

tattctgggg ggtggggtgg ggcaggacag caagggggag gattgggaag acaatagcag 1740

gcatgctggg gatgcggtgg gctctatggc ttctgaggcg gaaagaacca gtggcggtaa 1800

tacggttatc cacagaatca ggggataacg caggaaagaa catgtgagca aaaggccagc 1860

aaaaggccag gaaccgtaaa aaggccgcgt tgctggcgtt tttccatagg ctccgccccc 1920

ctgacgagca tcacaaaaat cgacgctcaa gtcagaggtg gcgaaacccg acaggactat 1980

aaagatacca ggcgtttccc cctggaagct ccctcgtgcg ctctcctgtt ccgaccctgc 2040

cgcttaccgg atacctgtcc gcctttctcc cttcgggaag cgtggcgctt tctcatagct 2100

cacgctgtag gtatctcagt tcggtgtagg tcgttcgctc caagctgggc tgtgtgcacg 2160

aaccccccgt tcagcccgac cgctgcgcct tatccggtaa ctatcgtctt gagtccaacc 2220

cggtaagaca cgacttatcg ccactggcag cagccactgg taacaggatt agcagagcga 2280

ggtatgtagg cggtgctaca gagttcttga agtggtggcc taactacggc tacactagaa 2340

ggacagtatt tggtatctgc gctctgctga agccagttac cttcggaaaa agagttggta 2400

gctcttgatc cggcaaacaa accaccgctg gtagcggtgg tttttttgtt tgcaagcagc 2460

agattacgcg cagaaaaaaa ggatctcaag aagatccttt gatcttttct acggggtctg 2520

acgctcagtg gaacgaaaac tcacgttaag ggattttggt catgagatta tcaaaaagga 2580

tcttcaccta gatcctttta aattaaaaat gaagttttaa atcaatctaa agtatatatg 2640

agtaacctga ggctatggca gggcctgccg ccccgacgtt ggctgcgagc cctgggcctt 2700

cacccgaact tggggggtgg ggtggggaaa aggaagaaac gcgggcgtat tggccccaat 2760

ggggtctcgg tggggtatcg acagagtgcc agccctggga ccgaaccccg cgtttatgaa 2820

caaacgaccc aacaccgtgc gttttattct gtctttttat tgccgtcata gcgcgggttc 2880

cttccggtat tgtctccttc cgtgtttcag ttagcctccc cctagggtgg gcgaagaact 2940

ccagcatgag atccccgcgc tggaggatca tccagccggc gtcccggaaa acgattccga 3000

agcccaacct ttcatagaag gcggcggtgg aatcgaaatc tcgtgatggc aggttgggcg 3060

tcgcttggtc ggtcatttcg aaccccagag tcccgctcag aagaactcgt caagaaggcg 3120

atagaaggcg atgcgctgcg aatcgggagc ggcgataccg taaagcacga ggaagcggtc 3180

agcccattcg ccgccaagct cttcagcaat atcacgggta gccaacgcta tgtcctgata 3240

gcggtccgcc acacccagcc ggccacagtc gatgaatcca gaaaagcggc cattttccac 3300

catgatattc ggcaagcagg catcgccatg ggtcacgacg agatcctcgc cgtcgggcat 3360

gctcgccttg agcctggcga acagttcggc tggcgcgagc ccctgatgct cttgatcatc 3420

ctgatcgaca agaccggctt ccatccgagt acgtgctcgc tcgatgcgat gtttcgcttg 3480

gtggtcgaat gggcaggtag ccggatcaag cgtatgcagc cgccgcattg catcagccat 3540

gatggatact ttctcggcag gagcaaggtg agatgacagg agatcctgcc ccggcacttc 3600

gcccaatagc agccagtccc ttcccgcttc agtgacaacg tcgagcacag ctgcgcaagg 3660

aacgcccgtc gtggccagcc acgatagccg cgctgcctcg tcttgcagtt cattcagggc 3720

accggacagg tcggtcttga caaaaagaac cgggcgcccc tgcgctgaca gccggaacac 3780

ggcggcatca gagcagccga ttgtctgttg tgcccagtca tagccgaata gcctctccac 3840

ccaagcggcc ggagaacctg cgtgcaatcc atcttgttca atcatgcgaa acgatcctca 3900

tcctgtctct tgatcgatct ttgcaaaagc ctaggcctcc aaaaaagcct cctcactact 3960

tctggaatag ctcagaggcc gaggcggcct cggcctctgc ataaataaaa aaaattagtc 4020

agccatgggg cggagaatgg gcggaactgg gcggagttag gggcgggatg ggcggagtta 4080

ggggcgggac tatggttgct gactaattga gatgcatgct ttgcatactt ctgcctgctg 4140

gggagcctgg ggactttcca cacctggttg ctgactaatt gagatgcatg ctttgcatac 4200

ttctgcctgc tggggagcct ggggactttc cacaccctaa ctgacacaca ttccacagct 4260

ggttctttcc gcctcaggac tcttcctttt tcaataaatc aatctaaagt atatatgagt 4320

aaacttggtc tgacagttac caatgcttaa tcagtgaggc acctatctca gcgatctgtc 4380

tatttcgttc atccatagtt gcctgactcc ccgtcgtgta gataactacg atacgggagg 4440

gcttaccatc tggccccagt gctgcaatga taccgcgaga cccacgctca ccggctccag 4500

atttatcagc aataaaccag ccagccggaa gggccgagcg cagaagtggt cctgcaactt 4560

tatccgcctc catccagtct attaattgtt gccgggaagc tagagtaagt agttcgccag 4620

ttaatagttt gcgcaacgtt gttgccattg ctacaggcat cgtggtgtca cgctcgtcgt 4680

ttggtatggc ttcattcagc tccggttccc aacgatcaag gcgagttaca tgatccccca 4740

tgttgtgcaa aaaagcggtt agctccttcg gtcctccgat cgttgtcaga agtaagttgg 4800

ccgcagtgtt atcactcatg gttatggcag cactgcataa ttctcttact gtcatgccat 4860

ccgtaagatg cttttctgtg actggtgagt actcaaccaa gtcattctga gaatagtgta 4920

tgcggcgacc gagttgctct tgcccggcgt caatacggga taataccgcg ccacatagca 4980

gaactttaaa agtgctcatc attggaaaac gttcttcggg gcgaaaactc tcaaggatct 5040

taccgctgtt gagatccagt tcgatgtaac ccactcgtgc acccaactga tcttcagcat 5100

cttttacttt caccagcgtt tctgggtgag caaaaacagg aaggcaaaat gccgcaaaaa 5160

agggaataag ggcgacacgg aaatgttgaa tactcatact cttccttttt caatattatt 5220

gaagcattta tcagggttat tgtctcatga gcggatacat atttgaatgt atttagaaaa 5280

ataaacaaat aggggttccg cgcacatttc cccgaaaagt gccacctgac gcgccctgta 5340

gcggcgcatt aagcgcggcg ggtgtggtgg ttacgcgcag cgtgaccgct acacttgcca 5400

gcgccctagc gcccgctcct ttcgctttct tcccttcctt tctcgccacg ttcgccggct 5460

ttccccgtca agctctaaat cgggggctcc ctttagggtt ccgatttagt gctttacggc 5520

acctcgaccc caaaaaactt gattagggtg atggttcacg tagtgggcca tcgccctgat 5580

agacggtttt tcgccctttg acgttggagt ccacgttctt taatagtgga ctcttgttcc 5640

aaactggaac aacactcaac cctatctcgg tctattcttt tgatttataa gggattttgc 5700

cgatttcggc ctattggtta aaaaatgagc tgatttaaca aaaatttaac gcgaatttta 5760

acaaaatatt aacgct 5776

<210> 12
<211> 5806
<212> DNA
<213> artificial

<220>
<223> Plasmid EAV_GP5_HA; BamH I / Xho I; Cloning of GP5 EAV + HA-tag

into pCR3.1

<220>
<221> misc_feature
<222> (682)..(1504)
<223> BamH I - Xho I ; EAV GP5 + HA-tag

<400> 12
gcgcgcgttg acattgatta ttgactagtt attaatagta atcaattacg gggtcattag 60

ttcatagccc atatatggag ttccgcgtta cataacttac ggtaaatggc ccgcctggct 120

gaccgcccaa cgacccccgc ccattgacgt caataatgac gtatgttccc atagtaacgc 180

caatagggac tttccattga cgtcaatggg tggactattt acggtaaact gcccacttgg 240

cagtacatca agtgtatcat atgccaagta cgccccctat tgacgtcaat gacggtaaat 300

ggcccgcctg gcattatgcc cagtacatga ccttatggga ctttcctact tggcagtaca 360

tctacgtatt agtcatcgct attaccatgg tgatgcggtt ttggcagtac atcaatgggc 420

gtggatagcg gtttgactca cggggatttc caagtctcca ccccattgac gtcaatggga 480

gtttgttttg gcaccaaaat caacgggact ttccaaaatg tcgtaacaac tccgccccat 540

tgacgcaaat gggcggtagg cgtgtacggt gggaggtcta tataagcaga gctctctggc 600

taactagaga acccactgct tactggctta tcgaaattaa tacgactcac tatagggaga 660

cccaagcttg gtaccgagct cggatccggt acgttgggct caacaatgtt atctatgatt 720

gtactgctat tcttgctttg gggtgcgcca tcacatgctt acttctcata ctacaccgct 780

cagcgcttca cagacttcac cttgtgtatg ctgacggatc gcggcgttat tgccaatttg 840

ctgcgatatg atgagcacac tgctttgtac aattgttccg ccagtaaaac ctgttggtat 900

tgcacattcc tggacgaaca gattatcacg tttggaaccg attgtaatga cacctacgcg 960

gtcccagttg ctgaggtcct ggaacaggcg catggaccgt acagtgtgct gtttaatgac 1020

atgccccctt ttatttacta tggccgtgaa ttcggcatag ttgtgttgga tgtgtttatg 1080

ttctatcccg ttttagttct gtttttctta tcagtactac cctatgctac gcttattctt 1140

gaaatgtgtg tatctattct gtttataatc tatggcattt acagcggggc ctacttggcc 1200

atgggcatat ttgcggccac gcttgctata cattcaattg tggtcctccg ccaattactg 1260

tggttatgcc tggcttggcg ataccgctgt acgcttcacg cgtcctttat atcagctgag 1320

gggaaagtgt accccgtaga ccccggactc ccggttgccg ccgcgggcaa tcggttgtta 1380

gtcccaggta ggcccactat cgattatgca gtggcctacg gcagcaaagt caaccttgtg 1440

aggttggggg cagctgaggt atgggagcca gcatacccct acgatgtacc cgattacgca 1500

tgactcgagc atgcatctag agggccctat tctatagtgt cacctaaatg ctagagctcg 1560

ctgatcagcc tcgactgtgc cttctagttg ccagccatct gttgtttgcc cctcccccgt 1620

gccttccttg accctggaag gtgccactcc cactgtcctt tcctaataaa atgaggaaat 1680

tgcatcgcat tgtctgagta ggtgtcattc tattctgggg ggtggggtgg ggcaggacag 1740

caagggggag gattgggaag acaatagcag gcatgctggg gatgcggtgg gctctatggc 1800

ttctgaggcg gaaagaacca gtggcggtaa tacggttatc cacagaatca ggggataacg 1860

caggaaagaa catgtgagca aaaggccagc aaaaggccag gaaccgtaaa aaggccgcgt 1920

tgctggcgtt tttccatagg ctccgccccc ctgacgagca tcacaaaaat cgacgctcaa 1980

gtcagaggtg gcgaaacccg acaggactat aaagatacca ggcgtttccc cctggaagct 2040

ccctcgtgcg ctctcctgtt ccgaccctgc cgcttaccgg atacctgtcc gcctttctcc 2100

cttcgggaag cgtggcgctt tctcatagct cacgctgtag gtatctcagt tcggtgtagg 2160

tcgttcgctc caagctgggc tgtgtgcacg aaccccccgt tcagcccgac cgctgcgcct 2220

tatccggtaa ctatcgtctt gagtccaacc cggtaagaca cgacttatcg ccactggcag 2280

cagccactgg taacaggatt agcagagcga ggtatgtagg cggtgctaca gagttcttga 2340

agtggtggcc taactacggc tacactagaa ggacagtatt tggtatctgc gctctgctga 2400

agccagttac cttcggaaaa agagttggta gctcttgatc cggcaaacaa accaccgctg 2460

gtagcggtgg tttttttgtt tgcaagcagc agattacgcg cagaaaaaaa ggatctcaag 2520

aagatccttt gatcttttct acggggtctg acgctcagtg gaacgaaaac tcacgttaag 2580

ggattttggt catgagatta tcaaaaagga tcttcaccta gatcctttta aattaaaaat 2640

gaagttttaa atcaatctaa agtatatatg agtaacctga ggctatggca gggcctgccg 2700

ccccgacgtt ggctgcgagc cctgggcctt cacccgaact tggggggtgg ggtggggaaa 2760

aggaagaaac gcgggcgtat tggccccaat ggggtctcgg tggggtatcg acagagtgcc 2820

agccctggga ccgaaccccg cgtttatgaa caaacgaccc aacaccgtgc gttttattct 2880

gtctttttat tgccgtcata gcgcgggttc cttccggtat tgtctccttc cgtgtttcag 2940

ttagcctccc cctagggtgg gcgaagaact ccagcatgag atccccgcgc tggaggatca 3000

tccagccggc gtcccggaaa acgattccga agcccaacct ttcatagaag gcggcggtgg 3060

aatcgaaatc tcgtgatggc aggttgggcg tcgcttggtc ggtcatttcg aaccccagag 3120

tcccgctcag aagaactcgt caagaaggcg atagaaggcg atgcgctgcg aatcgggagc 3180

ggcgataccg taaagcacga ggaagcggtc agcccattcg ccgccaagct cttcagcaat 3240

atcacgggta gccaacgcta tgtcctgata gcggtccgcc acacccagcc ggccacagtc 3300

gatgaatcca gaaaagcggc cattttccac catgatattc ggcaagcagg catcgccatg 3360

ggtcacgacg agatcctcgc cgtcgggcat gctcgccttg agcctggcga acagttcggc 3420

tggcgcgagc ccctgatgct cttgatcatc ctgatcgaca agaccggctt ccatccgagt 3480

acgtgctcgc tcgatgcgat gtttcgcttg gtggtcgaat gggcaggtag ccggatcaag 3540

cgtatgcagc cgccgcattg catcagccat gatggatact ttctcggcag gagcaaggtg 3600

agatgacagg agatcctgcc ccggcacttc gcccaatagc agccagtccc ttcccgcttc 3660

agtgacaacg tcgagcacag ctgcgcaagg aacgcccgtc gtggccagcc acgatagccg 3720

cgctgcctcg tcttgcagtt cattcagggc accggacagg tcggtcttga caaaaagaac 3780

cgggcgcccc tgcgctgaca gccggaacac ggcggcatca gagcagccga ttgtctgttg 3840

tgcccagtca tagccgaata gcctctccac ccaagcggcc ggagaacctg cgtgcaatcc 3900

atcttgttca atcatgcgaa acgatcctca tcctgtctct tgatcgatct ttgcaaaagc 3960

ctaggcctcc aaaaaagcct cctcactact tctggaatag ctcagaggcc gaggcggcct 4020

cggcctctgc ataaataaaa aaaattagtc agccatgggg cggagaatgg gcggaactgg 4080

gcggagttag gggcgggatg ggcggagtta ggggcgggac tatggttgct gactaattga 4140

gatgcatgct ttgcatactt ctgcctgctg gggagcctgg ggactttcca cacctggttg 4200

ctgactaatt gagatgcatg ctttgcatac ttctgcctgc tggggagcct ggggactttc 4260

cacaccctaa ctgacacaca ttccacagct ggttctttcc gcctcaggac tcttcctttt 4320

tcaataaatc aatctaaagt atatatgagt aaacttggtc tgacagttac caatgcttaa 4380

tcagtgaggc acctatctca gcgatctgtc tatttcgttc atccatagtt gcctgactcc 4440

ccgtcgtgta gataactacg atacgggagg gcttaccatc tggccccagt gctgcaatga 4500

taccgcgaga cccacgctca ccggctccag atttatcagc aataaaccag ccagccggaa 4560

gggccgagcg cagaagtggt cctgcaactt tatccgcctc catccagtct attaattgtt 4620

gccgggaagc tagagtaagt agttcgccag ttaatagttt gcgcaacgtt gttgccattg 4680

ctacaggcat cgtggtgtca cgctcgtcgt ttggtatggc ttcattcagc tccggttccc 4740

aacgatcaag gcgagttaca tgatccccca tgttgtgcaa aaaagcggtt agctccttcg 4800

gtcctccgat cgttgtcaga agtaagttgg ccgcagtgtt atcactcatg gttatggcag 4860

cactgcataa ttctcttact gtcatgccat ccgtaagatg cttttctgtg actggtgagt 4920

actcaaccaa gtcattctga gaatagtgta tgcggcgacc gagttgctct tgcccggcgt 4980

caatacggga taataccgcg ccacatagca gaactttaaa agtgctcatc attggaaaac 5040

gttcttcggg gcgaaaactc tcaaggatct taccgctgtt gagatccagt tcgatgtaac 5100

ccactcgtgc acccaactga tcttcagcat cttttacttt caccagcgtt tctgggtgag 5160

caaaaacagg aaggcaaaat gccgcaaaaa agggaataag ggcgacacgg aaatgttgaa 5220

tactcatact cttccttttt caatattatt gaagcattta tcagggttat tgtctcatga 5280

gcggatacat atttgaatgt atttagaaaa ataaacaaat aggggttccg cgcacatttc 5340

cccgaaaagt gccacctgac gcgccctgta gcggcgcatt aagcgcggcg ggtgtggtgg 5400

ttacgcgcag cgtgaccgct acacttgcca gcgccctagc gcccgctcct ttcgctttct 5460

tcccttcctt tctcgccacg ttcgccggct ttccccgtca agctctaaat cgggggctcc 5520

ctttagggtt ccgatttagt gctttacggc acctcgaccc caaaaaactt gattagggtg 5580

atggttcacg tagtgggcca tcgccctgat agacggtttt tcgccctttg acgttggagt 5640

ccacgttctt taatagtgga ctcttgttcc aaactggaac aacactcaac cctatctcgg 5700

tctattcttt tgatttataa gggattttgc cgatttcggc ctattggtta aaaaatgagc 5760

tgatttaaca aaaatttaac gcgaatttta acaaaatatt aacgct 5806

<210> 13
<211> 5803
<212> DNA
<213> Artificial

<220>
<223> Plasmid EAV_GP5_FLAG; BamH I / Xho I; Cloning of GP5 EAV +
FLAG-tag into pCR3.1

<220>
<221> misc_feature
<222> (682)..(1501)
<223> BamH I - Xho I ; EAV GP5 + FLAG-tag

<400> 13
gcgcgcgttg acattgatta ttgactagtt attaatagta atcaattacg gggtcattag 60

ttcatagccc atatatggag ttccgcgtta cataacttac ggtaaatggc ccgcctggct 120

gaccgcccaa cgacccccgc ccattgacgt caataatgac gtatgttccc atagtaacgc 180

caatagggac tttccattga cgtcaatggg tggactattt acggtaaact gcccacttgg 240

cagtacatca agtgtatcat atgccaagta cgccccctat tgacgtcaat gacggtaaat 300

ggcccgcctg gcattatgcc cagtacatga ccttatggga ctttcctact tggcagtaca 360

tctacgtatt agtcatcgct attaccatgg tgatgcggtt ttggcagtac atcaatgggc 420

gtggatagcg gtttgactca cggggatttc caagtctcca ccccattgac gtcaatggga 480

gtttgttttg gcaccaaaat caacgggact ttccaaaatg tcgtaacaac tccgccccat 540

tgacgcaaat gggcggtagg cgtgtacggt gggaggtcta tataagcaga gctctctggc 600

taactagaga acccactgct tactggctta tcgaaattaa tacgactcac tatagggaga 660

cccaagcttg gtaccgagct cggatccggt acgttgggct caacaatgtt atctatgatt 720

gtactgctat tcttgctttg gggtgcgcca tcacatgctt acttctcata ctacaccgct 780

cagcgcttca cagacttcac cttgtgtatg ctgacggatc gcggcgttat tgccaatttg 840

ctgcgatatg atgagcacac tgctttgtac aattgttccg ccagtaaaac ctgttggtat 900

tgcacattcc tggacgaaca gattatcacg tttggaaccg attgtaatga cacctacgcg 960

gtcccagttg ctgaggtcct ggaacaggcg catggaccgt acagtgtgct gtttaatgac 1020

atgccccctt ttatttacta tggccgtgaa ttcggcatag ttgtgttgga tgtgtttatg 1080

ttctatcccg ttttagttct gtttttctta tcagtactac cctatgctac gcttattctt 1140

gaaatgtgtg tatctattct gtttataatc tatggcattt acagcggggc ctacttggcc 1200

atgggcatat ttgcggccac gcttgctata cattcaattg tggtcctccg ccaattactg 1260

tggttatgcc tggcttggcg ataccgctgt acgcttcacg cgtcctttat atcagctgag 1320

gggaaagtgt accccgtaga ccccggactc ccggttgccg ccgcgggcaa tcggttgtta 1380

gtcccaggta ggcccactat cgattatgca gtggcctacg gcagcaaagt caaccttgtg 1440

aggttggggg cagctgaggt atgggagcca gcagactaca aagacgatga cgataagtga 1500

ctcgagcatg catctagagg gccctattct atagtgtcac ctaaatgcta gagctcgctg 1560

atcagcctcg actgtgcctt ctagttgcca gccatctgtt gtttgcccct cccccgtgcc 1620

ttccttgacc ctggaaggtg ccactcccac tgtcctttcc taataaaatg aggaaattgc 1680

atcgcattgt ctgagtaggt gtcattctat tctggggggt ggggtggggc aggacagcaa 1740

gggggaggat tgggaagaca atagcaggca tgctggggat gcggtgggct ctatggcttc 1800

tgaggcggaa agaaccagtg gcggtaatac ggttatccac agaatcaggg gataacgcag 1860

gaaagaacat gtgagcaaaa ggccagcaaa aggccaggaa ccgtaaaaag gccgcgttgc 1920

tggcgttttt ccataggctc cgcccccctg acgagcatca caaaaatcga cgctcaagtc 1980

agaggtggcg aaacccgaca ggactataaa gataccaggc gtttccccct ggaagctccc 2040

tcgtgcgctc tcctgttccg accctgccgc ttaccggata cctgtccgcc tttctccctt 2100

cgggaagcgt ggcgctttct catagctcac gctgtaggta tctcagttcg gtgtaggtcg 2160

ttcgctccaa gctgggctgt gtgcacgaac cccccgttca gcccgaccgc tgcgccttat 2220

ccggtaacta tcgtcttgag tccaacccgg taagacacga cttatcgcca ctggcagcag 2280

ccactggtaa caggattagc agagcgaggt atgtaggcgg tgctacagag ttcttgaagt 2340

ggtggcctaa ctacggctac actagaagga cagtatttgg tatctgcgct ctgctgaagc 2400

cagttacctt cggaaaaaga gttggtagct cttgatccgg caaacaaacc accgctggta 2460

gcggtggttt ttttgtttgc aagcagcaga ttacgcgcag aaaaaaagga tctcaagaag 2520

atcctttgat cttttctacg gggtctgacg ctcagtggaa cgaaaactca cgttaaggga 2580

ttttggtcat gagattatca aaaaggatct tcacctagat ccttttaaat taaaaatgaa 2640

gttttaaatc aatctaaagt atatatgagt aacctgaggc tatggcaggg cctgccgccc 2700

cgacgttggc tgcgagccct gggccttcac ccgaacttgg ggggtggggt ggggaaaagg 2760

aagaaacgcg ggcgtattgg ccccaatggg gtctcggtgg ggtatcgaca gagtgccagc 2820

cctgggaccg aaccccgcgt ttatgaacaa acgacccaac accgtgcgtt ttattctgtc 2880

tttttattgc cgtcatagcg cgggttcctt ccggtattgt ctccttccgt gtttcagtta 2940

gcctccccct agggtgggcg aagaactcca gcatgagatc cccgcgctgg aggatcatcc 3000

agccggcgtc ccggaaaacg attccgaagc ccaacctttc atagaaggcg gcggtggaat 3060

cgaaatctcg tgatggcagg ttgggcgtcg cttggtcggt catttcgaac cccagagtcc 3120

cgctcagaag aactcgtcaa gaaggcgata gaaggcgatg cgctgcgaat cgggagcggc 3180

gataccgtaa agcacgagga agcggtcagc ccattcgccg ccaagctctt cagcaatatc 3240

acgggtagcc aacgctatgt cctgatagcg gtccgccaca cccagccggc cacagtcgat 3300

gaatccagaa aagcggccat tttccaccat gatattcggc aagcaggcat cgccatgggt 3360

cacgacgaga tcctcgccgt cgggcatgct cgccttgagc ctggcgaaca gttcggctgg 3420

cgcgagcccc tgatgctctt gatcatcctg atcgacaaga ccggcttcca tccgagtacg 3480

tgctcgctcg atgcgatgtt tcgcttggtg gtcgaatggg caggtagccg gatcaagcgt 3540

atgcagccgc cgcattgcat cagccatgat ggatactttc tcggcaggag caaggtgaga 3600

tgacaggaga tcctgccccg gcacttcgcc caatagcagc cagtcccttc ccgcttcagt 3660

gacaacgtcg agcacagctg cgcaaggaac gcccgtcgtg gccagccacg atagccgcgc 3720

tgcctcgtct tgcagttcat tcagggcacc ggacaggtcg gtcttgacaa aaagaaccgg 3780

gcgcccctgc gctgacagcc ggaacacggc ggcatcagag cagccgattg tctgttgtgc 3840

ccagtcatag ccgaatagcc tctccaccca agcggccgga gaacctgcgt gcaatccatc 3900

ttgttcaatc atgcgaaacg atcctcatcc tgtctcttga tcgatctttg caaaagccta 3960

ggcctccaaa aaagcctcct cactacttct ggaatagctc agaggccgag gcggcctcgg 4020

cctctgcata aataaaaaaa attagtcagc catggggcgg agaatgggcg gaactgggcg 4080

gagttagggg cgggatgggc ggagttaggg gcgggactat ggttgctgac taattgagat 4140

gcatgctttg catacttctg cctgctgggg agcctgggga ctttccacac ctggttgctg 4200

actaattgag atgcatgctt tgcatacttc tgcctgctgg ggagcctggg gactttccac 4260

accctaactg acacacattc cacagctggt tctttccgcc tcaggactct tcctttttca 4320

ataaatcaat ctaaagtata tatgagtaaa cttggtctga cagttaccaa tgcttaatca 4380

gtgaggcacc tatctcagcg atctgtctat ttcgttcatc catagttgcc tgactccccg 4440

tcgtgtagat aactacgata cgggagggct taccatctgg ccccagtgct gcaatgatac 4500

cgcgagaccc acgctcaccg gctccagatt tatcagcaat aaaccagcca gccggaaggg 4560

ccgagcgcag aagtggtcct gcaactttat ccgcctccat ccagtctatt aattgttgcc 4620

gggaagctag agtaagtagt tcgccagtta atagtttgcg caacgttgtt gccattgcta 4680

caggcatcgt ggtgtcacgc tcgtcgtttg gtatggcttc attcagctcc ggttcccaac 4740

gatcaaggcg agttacatga tcccccatgt tgtgcaaaaa agcggttagc tccttcggtc 4800

ctccgatcgt tgtcagaagt aagttggccg cagtgttatc actcatggtt atggcagcac 4860

tgcataattc tcttactgtc atgccatccg taagatgctt ttctgtgact ggtgagtact 4920

caaccaagtc attctgagaa tagtgtatgc ggcgaccgag ttgctcttgc ccggcgtcaa 4980

tacgggataa taccgcgcca catagcagaa ctttaaaagt gctcatcatt ggaaaacgtt 5040

cttcggggcg aaaactctca aggatcttac cgctgttgag atccagttcg atgtaaccca 5100

ctcgtgcacc caactgatct tcagcatctt ttactttcac cagcgtttct gggtgagcaa 5160

aaacaggaag gcaaaatgcc gcaaaaaagg gaataagggc gacacggaaa tgttgaatac 5220

tcatactctt cctttttcaa tattattgaa gcatttatca gggttattgt ctcatgagcg 5280

gatacatatt tgaatgtatt tagaaaaata aacaaatagg ggttccgcgc acatttcccc 5340

gaaaagtgcc acctgacgcg ccctgtagcg gcgcattaag cgcggcgggt gtggtggtta 5400

cgcgcagcgt gaccgctaca cttgccagcg ccctagcgcc cgctcctttc gctttcttcc 5460

cttcctttct cgccacgttc gccggctttc cccgtcaagc tctaaatcgg gggctccctt 5520

tagggttccg atttagtgct ttacggcacc tcgaccccaa aaaacttgat tagggtgatg 5580

gttcacgtag tgggccatcg ccctgataga cggtttttcg ccctttgacg ttggagtcca 5640

cgttctttaa tagtggactc ttgttccaaa ctggaacaac actcaaccct atctcggtct 5700

attcttttga tttataaggg attttgccga tttcggccta ttggttaaaa aatgagctga 5760

tttaacaaaa atttaacgcg aattttaaca aaatattaac gct 5803

<210> 14
<211> 5704
<212> DNA
<213> Artificial

<220>
<223> Plasmid GP5_lely_PCV2; Hind III / Xho I; Cloning of GP5 PRRSV
Lelystad + PCV2 neutralizing epitope into pCR3.1

<220>
<221> misc_feature
<222> (664)..(1402)
<223> Hind III - Xho I ; PRRSV lelystad GP5 + PCV2 neutralizing epitope

<400> 14
gcgcgcgttg acattgatta ttgactagtt attaatagta atcaattacg gggtcattag 60

ttcatagccc atatatggag ttccgcgtta cataacttac ggtaaatggc ccgcctggct 120

gaccgcccaa cgacccccgc ccattgacgt caataatgac gtatgttccc atagtaacgc 180

caatagggac tttccattga cgtcaatggg tggactattt acggtaaact gcccacttgg 240

cagtacatca agtgtatcat atgccaagta cgccccctat tgacgtcaat gacggtaaat 300

ggcccgcctg gcattatgcc cagtacatga ccttatggga ctttcctact tggcagtaca 360

tctacgtatt agtcatcgct attaccatgg tgatgcggtt ttggcagtac atcaatgggc 420

gtggatagcg gtttgactca cggggatttc caagtctcca ccccattgac gtcaatggga 480

gtttgttttg gcaccaaaat caacgggact ttccaaaatg tcgtaacaac tccgccccat 540

tgacgcaaat gggcggtagg cgtgtacggt gggaggtcta tataagcaga gctctctggc 600

taactagaga acccactgct tactggctta tcgaaattaa tacgactcac tatagggaga 660

cccaagctta ccatgagatg ttctcacaaa ttggggcgtt tcttgactcc gcactcttgc 720

ttctggtggt tttttttgct gtgtaccggc ttgtcctggt cctttgccga tggcaacggc 780

aacagctcga cataccaata catatataac ttgacgatat gcgagctgaa tgggaccgac 840

tggttgtcca gccattttgg ttgggcagtc gagacctttg tgttttaccc ggttgccact 900

catatcctct cactgggttt tctcacaaca agccattttt ttgacgcgct cggtctcggc 960

gctgtatcca ctgcaggatt tgttggcggg cggtatgtac tctgcagcgt ctacggcgct 1020

tgtgctttcg cagcgttcgt atgttttgtc atccgtgctg ctaaaaattg catggcctgc 1080

cgctatgccc gtacccggtt taccaacttc attgtagaca accgggggag agttcatcga 1140

tggaagtctc caatagtggt agaaaaattg ggcaaagccg aagtcgacgg caacctcgtc 1200

accatcaaac atgtcgtcct cgaaggggtt aaagctcaac ccttgacgag gacttcggct 1260

gagcaatggg aggccgcagg atccggagca aaacccgtgt tggactccac tatcgactac 1320

tttcagccga acaacaagcg gaaccaactg tggctgcgac tgcagaccgc tggcaatgtc 1380

gatcacgttg gtctgggata actcgagcat gcatctagag ggccctattc tatagtgtca 1440

cctaaatgct agagctcgct gatcagcctc gactgtgcct tctagttgcc agccatctgt 1500

tgtttgcccc tcccccgtgc cttccttgac cctggaaggt gccactccca ctgtcctttc 1560

ctaataaaat gaggaaattg catcgcattg tctgagtagg tgtcattcta ttctgggggg 1620

tggggtgggg caggacagca agggggagga ttgggaagac aatagcaggc atgctgggga 1680

tgcggtgggc tctatggctt ctgaggcgga aagaaccagt ggcggtaata cggttatcca 1740

cagaatcagg ggataacgca ggaaagaaca tgtgagcaaa aggccagcaa aaggccagga 1800

accgtaaaaa ggccgcgttg ctggcgtttt tccataggct ccgcccccct gacgagcatc 1860

acaaaaatcg acgctcaagt cagaggtggc gaaacccgac aggactataa agataccagg 1920

cgtttccccc tggaagctcc ctcgtgcgct ctcctgttcc gaccctgccg cttaccggat 1980

acctgtccgc ctttctccct tcgggaagcg tggcgctttc tcatagctca cgctgtaggt 2040

atctcagttc ggtgtaggtc gttcgctcca agctgggctg tgtgcacgaa ccccccgttc 2100

agcccgaccg ctgcgcctta tccggtaact atcgtcttga gtccaacccg gtaagacacg 2160

acttatcgcc actggcagca gccactggta acaggattag cagagcgagg tatgtaggcg 2220

gtgctacaga gttcttgaag tggtggccta actacggcta cactagaagg acagtatttg 2280

gtatctgcgc tctgctgaag ccagttacct tcggaaaaag agttggtagc tcttgatccg 2340

gcaaacaaac caccgctggt agcggtggtt tttttgtttg caagcagcag attacgcgca 2400

gaaaaaaagg atctcaagaa gatcctttga tcttttctac ggggtctgac gctcagtgga 2460

acgaaaactc acgttaaggg attttggtca tgagattatc aaaaaggatc ttcacctaga 2520

tccttttaaa ttaaaaatga agttttaaat caatctaaag tatatatgag taacctgagg 2580

ctatggcagg gcctgccgcc ccgacgttgg ctgcgagccc tgggccttca cccgaacttg 2640

gggggtgggg tggggaaaag gaagaaacgc gggcgtattg gccccaatgg ggtctcggtg 2700

gggtatcgac agagtgccag ccctgggacc gaaccccgcg tttatgaaca aacgacccaa 2760

caccgtgcgt tttattctgt ctttttattg ccgtcatagc gcgggttcct tccggtattg 2820

tctccttccg tgtttcagtt agcctccccc tagggtgggc gaagaactcc agcatgagat 2880

ccccgcgctg gaggatcatc cagccggcgt cccggaaaac gattccgaag cccaaccttt 2940

catagaaggc ggcggtggaa tcgaaatctc gtgatggcag gttgggcgtc gcttggtcgg 3000

tcatttcgaa ccccagagtc ccgctcagaa gaactcgtca agaaggcgat agaaggcgat 3060

gcgctgcgaa tcgggagcgg cgataccgta aagcacgagg aagcggtcag cccattcgcc 3120

gccaagctct tcagcaatat cacgggtagc caacgctatg tcctgatagc ggtccgccac 3180

acccagccgg ccacagtcga tgaatccaga aaagcggcca ttttccacca tgatattcgg 3240

caagcaggca tcgccatggg tcacgacgag atcctcgccg tcgggcatgc tcgccttgag 3300

cctggcgaac agttcggctg gcgcgagccc ctgatgctct tgatcatcct gatcgacaag 3360

accggcttcc atccgagtac gtgctcgctc gatgcgatgt ttcgcttggt ggtcgaatgg 3420

gcaggtagcc ggatcaagcg tatgcagccg ccgcattgca tcagccatga tggatacttt 3480

ctcggcagga gcaaggtgag atgacaggag atcctgcccc ggcacttcgc ccaatagcag 3540

ccagtccctt cccgcttcag tgacaacgtc gagcacagct gcgcaaggaa cgcccgtcgt 3600

ggccagccac gatagccgcg ctgcctcgtc ttgcagttca ttcagggcac cggacaggtc 3660

ggtcttgaca aaaagaaccg ggcgcccctg cgctgacagc cggaacacgg cggcatcaga 3720

gcagccgatt gtctgttgtg cccagtcata gccgaatagc ctctccaccc aagcggccgg 3780

agaacctgcg tgcaatccat cttgttcaat catgcgaaac gatcctcatc ctgtctcttg 3840

atcgatcttt gcaaaagcct aggcctccaa aaaagcctcc tcactacttc tggaatagct 3900

cagaggccga ggcggcctcg gcctctgcat aaataaaaaa aattagtcag ccatggggcg 3960

gagaatgggc ggaactgggc ggagttaggg gcgggatggg cggagttagg ggcgggacta 4020

tggttgctga ctaattgaga tgcatgcttt gcatacttct gcctgctggg gagcctgggg 4080

actttccaca cctggttgct gactaattga gatgcatgct ttgcatactt ctgcctgctg 4140

gggagcctgg ggactttcca caccctaact gacacacatt ccacagctgg ttctttccgc 4200

ctcaggactc ttcctttttc aataaatcaa tctaaagtat atatgagtaa acttggtctg 4260

acagttacca atgcttaatc agtgaggcac ctatctcagc gatctgtcta tttcgttcat 4320

ccatagttgc ctgactcccc gtcgtgtaga taactacgat acgggagggc ttaccatctg 4380

gccccagtgc tgcaatgata ccgcgagacc cacgctcacc ggctccagat ttatcagcaa 4440

taaaccagcc agccggaagg gccgagcgca gaagtggtcc tgcaacttta tccgcctcca 4500

tccagtctat taattgttgc cgggaagcta gagtaagtag ttcgccagtt aatagtttgc 4560

gcaacgttgt tgccattgct acaggcatcg tggtgtcacg ctcgtcgttt ggtatggctt 4620

cattcagctc cggttcccaa cgatcaaggc gagttacatg atcccccatg ttgtgcaaaa 4680

aagcggttag ctccttcggt cctccgatcg ttgtcagaag taagttggcc gcagtgttat 4740

cactcatggt tatggcagca ctgcataatt ctcttactgt catgccatcc gtaagatgct 4800

tttctgtgac tggtgagtac tcaaccaagt cattctgaga atagtgtatg cggcgaccga 4860

gttgctcttg cccggcgtca atacgggata ataccgcgcc acatagcaga actttaaaag 4920

tgctcatcat tggaaaacgt tcttcggggc gaaaactctc aaggatctta ccgctgttga 4980

gatccagttc gatgtaaccc actcgtgcac ccaactgatc ttcagcatct tttactttca 5040

ccagcgtttc tgggtgagca aaaacaggaa ggcaaaatgc cgcaaaaaag ggaataaggg 5100

cgacacggaa atgttgaata ctcatactct tcctttttca atattattga agcatttatc 5160

agggttattg tctcatgagc ggatacatat ttgaatgtat ttagaaaaat aaacaaatag 5220

gggttccgcg cacatttccc cgaaaagtgc cacctgacgc gccctgtagc ggcgcattaa 5280

gcgcggcggg tgtggtggtt acgcgcagcg tgaccgctac acttgccagc gccctagcgc 5340

ccgctccttt cgctttcttc ccttcctttc tcgccacgtt cgccggcttt ccccgtcaag 5400

ctctaaatcg ggggctccct ttagggttcc gatttagtgc tttacggcac ctcgacccca 5460

aaaaacttga ttagggtgat ggttcacgta gtgggccatc gccctgatag acggtttttc 5520

gccctttgac gttggagtcc acgttcttta atagtggact cttgttccaa actggaacaa 5580

cactcaaccc tatctcggtc tattcttttg atttataagg gattttgccg atttcggcct 5640

attggttaaa aaatgagctg atttaacaaa aatttaacgc gaattttaac aaaatattaa 5700

cgct 5704

<210> 15
<211> 242
<212> PRT
<213> Artificial

<220>

<223>  PRRSV lelystad GP5 fused to PCV2 neutralizing epitope

<400>  15

Met Arg Cys Ser His Lys Leu Gly Arg Phe Leu Thr Pro His Ser Cys
1               5                   10                  15

Phe Trp Trp Phe Phe Leu Leu Cys Thr Gly Leu Ser Trp Ser Phe Ala
            20                  25                  30

Asp Gly Asn Gly Asn Ser Ser Thr Tyr Gln Tyr Ile Tyr Asn Leu Thr
        35                  40                  45

Ile Cys Glu Leu Asn Gly Thr Asp Trp Leu Ser Ser His Phe Gly Trp
    50                  55                  60

Ala Val Glu Thr Phe Val Phe Tyr Pro Val Ala Thr His Ile Leu Ser
65                  70                  75                  80

Leu Gly Phe Leu Thr Thr Ser His Phe Phe Asp Ala Leu Gly Leu Gly
                85                  90                  95

Ala Val Ser Thr Ala Gly Phe Val Gly Gly Arg Tyr Val Leu Cys Ser
            100                 105                 110

Val Tyr Gly Ala Cys Ala Phe Ala Ala Phe Val Cys Phe Val Ile Arg
        115                 120                 125

Ala Ala Lys Asn Cys Met Ala Cys Arg Tyr Ala Arg Thr Arg Phe Thr
    130                 135                 140

Asn Phe Ile Val Asp Asn Arg Gly Arg Val His Arg Trp Lys Ser Pro
145                 150                 155                 160

Ile Val Val Glu Lys Leu Gly Lys Ala Glu Val Asp Gly Asn Leu Val
                165                 170                 175

Thr Ile Lys His Val Val Leu Glu Gly Val Lys Ala Gln Pro Leu Thr
            180                 185                 190

Arg Thr Ser Ala Glu Gln Trp Glu Ala Ala Gly Ser Gly Ala Lys Pro
        195                 200                 205

Val Leu Asp Ser Thr Ile Asp Tyr Phe Gln Pro Asn Asn Lys Arg Asn
    210                 215                 220

Gln Leu Trp Leu Arg Leu Gln Thr Ala Gly Asn Val Asp His Val Gly
225                 230                 235                 240

Leu Gly

<210> 16
<211> 264
<212> PRT
<213> Artificial

<220>
<223> EAV GP5 fused to FLAG-tag

<400> 16

Met Leu Ser Met Ile Val Leu Leu Phe Leu Leu Trp Gly Ala Pro Ser
1               5                   10                  15

His Ala Tyr Phe Ser Tyr Tyr Thr Ala Gln Arg Phe Thr Asp Phe Thr
            20                  25                  30

Leu Cys Met Leu Thr Asp Arg Gly Val Ile Ala Asn Leu Leu Arg Tyr
        35                  40                  45

Asp Glu His Thr Ala Leu Tyr Asn Cys Ser Ala Ser Lys Thr Cys Trp
    50                  55                  60

Tyr Cys Thr Phe Leu Asp Glu Gln Ile Ile Thr Phe Gly Thr Asp Cys
65                  70                  75                  80

Asn Asp Thr Tyr Ala Val Pro Val Ala Glu Val Leu Glu Gln Ala His
                85                  90                  95

Gly Pro Tyr Ser Val Leu Phe Asn Asp Met Pro Pro Phe Ile Tyr Tyr
            100                 105                 110

Gly Arg Glu Phe Gly Ile Val Val Leu Asp Val Phe Met Phe Tyr Pro
        115                 120                 125

Val Leu Val Leu Phe Phe Leu Ser Val Leu Pro Tyr Ala Thr Leu Ile
    130                 135                 140

Leu Glu Met Cys Val Ser Ile Leu Phe Ile Ile Tyr Gly Ile Tyr Ser
145                 150                 155                 160

Gly Ala Tyr Leu Ala Met Gly Ile Phe Ala Ala Thr Leu Ala Ile His
                165                 170                 175

Ser Ile Val Val Leu Arg Gln Leu Leu Trp Leu Cys Leu Ala Trp Arg
            180                 185                 190

Tyr Arg Cys Thr Leu His Ala Ser Phe Ile Ser Ala Glu Gly Lys Val
      195                 200                 205

Tyr Pro Val Asp Pro Gly Leu Pro Val Ala Ala Ala Gly Asn Arg Leu
    210                 215                 220

Leu Val Pro Gly Arg Pro Thr Ile Asp Tyr Ala Val Ala Tyr Gly Ser
225                 230                 235                 240

Lys Val Asn Leu Val Arg Leu Gly Ala Ala Glu Val Trp Glu Pro Ala
                245                 250                 255

Asp Tyr Lys Asp Asp Asp Asp Lys
            260

<210>  17
<211>  5809
<212>  DNA
<213>  artificial

<220>
<223>  Plasmid GP3_HA; BamH I / Xba I; Cloning of GP3 PRRSV
       Lelystad + HA-tag into pCR3.1

<220>
<221>  misc_feature
<222>  (682)..(1519)
<223>  BamH I - Xba I ; PRRSV lelystad GP3 + HA-tag

<400>  17
gcgcgcgttg acattgatta ttgactagtt attaatagta atcaattacg gggtcattag      60

ttcatagccc atatatggag ttccgcgtta cataacttac ggtaaatggc ccgcctggct     120

gaccgcccaa cgacccccgc ccattgacgt caataatgac gtatgttccc atagtaacgc     180

caatagggac tttccattga cgtcaatggg tggactattt acggtaaact gcccacttgg     240

cagtacatca agtgtatcat atgccaagta cgccccctat tgacgtcaat gacggtaaat     300

ggcccgcctg gcattatgcc cagtacatga ccttatggga ctttcctact tggcagtaca     360

tctacgtatt agtcatcgct attaccatgg tgatgcggtt ttggcagtac atcaatgggc     420

gtggatagcg gtttgactca cggggatttc caagtctcca ccccattgac gtcaatggga     480

gtttgttttg gcaccaaaat caacgggact ttccaaaatg tcgtaacaac tccgccccat     540

tgacgcaaat gggcggtagg cgtgtacggt gggaggtcta tataagcaga gctctctggc     600

taactagaga acccactgct tactggctta tcgaaattaa tacgactcac tatagggaga     660

cccaagcttg gtaccgagct cggatccaca atggctcatc agtgtgcacg cttccatttt     720

ttcctctgtg gcttcatctg ttaccttgtt catagtgctt tggcttcgaa ttccagctct     780

acgctatgtt tttggtttcc attggcccac ggcaacacat cattcgagct gaccatcaac     840

tacaccatat gcatgccctg ttctaccagt caagcggctc gccaaaggct cgagcccggt 900
cgtaacatgt ggtgcaaaat agggtatgac aggtgtgagg agcgtgacca tgatgagttg 960
ttaatgccca tcccgtccgg gtacgacaac ctcaaacttg agggttatta tgcttggctg 1020
gcttttttgt cctttcctta cgcggcccaa ttccatccgg agttgttcgg gatagggaat 1080
gtgtcgcgcg tcttcgtgga caagcgacac cagttcattt gtgccgagca tgatggacag 1140
aattcaaccg tatctaccgg acacaacatc tccgcattat atgcggcata ttaccaccac 1200
caaatagacg ggggcaattg gttccatttg gaatggctgc ggccactctt ttcctcctgg 1260
ctggtgctca atatatcatg gtttctgagg cgttcgcctg taagccctgt ttctcgacgc 1320
atctatcaga tattgagacc aacacgaccg cggctgccgg tttcatggtc cttcaggaca 1380
tcaattgtct ccgacctcac ggggtctcag cagcgcaaga ggaaattccc ttcggaaagt 1440
cgtcccaatg tcgtgaagcc gtcggtactc cccagtacat cacgagcata cccctacgat 1500
gtacccgatt acgcatgatc tagagggccc tattctatag tgtcacctaa atgctagagc 1560
tcgctgatca gcctcgactg tgccttctag ttgccagcca tctgttgttt gcccctcccc 1620
cgtgccttcc ttgaccctgg aaggtgccac tcccactgtc ctttcctaat aaaatgagga 1680
aattgcatcg cattgtctga gtaggtgtca ttctattctg gggggtgggg tggggcagga 1740
cagcaagggg gaggattggg aagacaatag caggcatgct ggggatgcgg tgggctctat 1800
ggcttctgag gcggaaagaa ccagtggcgg taatacggtt atccacagaa tcaggggata 1860
acgcaggaaa gaacatgtga gcaaaaggcc agcaaaaggc caggaaccgt aaaaaggccg 1920
cgttgctggc gtttttccat aggctccgcc cccctgacga gcatcacaaa aatcgacgct 1980
caagtcagag gtggcgaaac ccgacaggac tataaagata ccaggcgttt ccccctggaa 2040
gctccctcgt gcgctctcct gttccgaccc tgccgcttac cggatacctg tccgcctttc 2100
tcccttcggg aagcgtggcg ctttctcata gctcacgctg taggtatctc agttcggtgt 2160
aggtcgttcg ctccaagctg ggctgtgtgc acgaaccccc cgttcagccc gaccgctgcg 2220
ccttatccgg taactatcgt cttgagtcca acccggtaag acacgactta tcgccactgg 2280
cagcagccac tggtaacagg attagcagag cgaggtatgt aggcggtgct acagagttct 2340
tgaagtggtg gcctaactac ggctacacta gaaggacagt atttggtatc tgcgctctgc 2400
tgaagccagt taccttcgga aaaagagttg gtagctcttg atccggcaaa caaaccaccg 2460
ctggtagcgg tggttttttt gtttgcaagc agcagattac gcgcagaaaa aaaggatctc 2520
aagaagatcc tttgatcttt tctacggggt ctgacgctca gtggaacgaa aactcacgtt 2580
aagggatttt ggtcatgaga ttatcaaaaa ggatcttcac ctagatcctt ttaaattaaa 2640
aatgaagttt taaatcaatc taaagtatat atgagtaacc tgaggctatg gcagggcctg 2700

ccgccccgac gttggctgcg agccctgggc cttcacccga acttgggggg tggggtgggg 2760

aaaaggaaga aacgcgggcg tattggcccc aatggggtct cggtggggta tcgacagagt 2820

gccagccctg ggaccgaacc ccgcgtttat gaacaaacga cccaacaccg tgcgttttat 2880

tctgtctttt tattgccgtc atagcgcggg ttccttccgg tattgtctcc ttccgtgttt 2940

cagttagcct ccccctaggg tgggcgaaga actccagcat gagatccccg cgctggagga 3000

tcatccagcc ggcgtcccgg aaaacgattc cgaagcccaa cctttcatag aaggcggcgg 3060

tggaatcgaa atctcgtgat ggcaggttgg gcgtcgcttg gtcggtcatt tcgaacccca 3120

gagtcccgct cagaagaact cgtcaagaag gcgatagaag gcgatgcgct gcgaatcggg 3180

agcggcgata ccgtaaagca cgaggaagcg gtcagcccat tcgccgccaa gctcttcagc 3240

aatatcacgg gtagccaacg ctatgtcctg atagcggtcc gccacaccca gccggccaca 3300

gtcgatgaat ccagaaaagc ggccattttc caccatgata ttcggcaagc aggcatcgcc 3360

atgggtcacg acgagatcct cgccgtcggg catgctcgcc ttgagcctgg cgaacagttc 3420

ggctggcgcg agcccctgat gctcttgatc atcctgatcg acaagaccgg cttccatccg 3480

agtacgtgct cgctcgatgc gatgtttcgc ttggtggtcg aatgggcagg tagccggatc 3540

aagcgtatgc agccgccgca ttgcatcagc catgatggat actttctcgg caggagcaag 3600

gtgagatgac aggagatcct gccccggcac ttcgcccaat agcagccagt cccttcccgc 3660

ttcagtgaca acgtcgagca cagctgcgca aggaacgccc gtcgtggcca gccacgatag 3720

ccgcgctgcc tcgtcttgca gttcattcag ggcaccggac aggtcggtct tgacaaaaag 3780

aaccgggcgc ccctgcgctg acagccggaa cacggcggca tcagagcagc cgattgtctg 3840

ttgtgcccag tcatagccga atagcctctc cacccaagcg gccggagaac ctgcgtgcaa 3900

tccatcttgt tcaatcatgc gaaacgatcc tcatcctgtc tcttgatcga tctttgcaaa 3960

agcctaggcc tccaaaaaag cctcctcact acttctggaa tagctcagag gccgaggcgg 4020

cctcggcctc tgcataaata aaaaaaatta gtcagccatg gggcggagaa tgggcggaac 4080

tgggcggagt taggggcggg atgggcggag ttaggggcgg gactatggtt gctgactaat 4140

tgagatgcat gctttgcata cttctgcctg ctggggagcc tggggacttt ccacacctgg 4200

ttgctgacta attgagatgc atgctttgca tacttctgcc tgctggggag cctggggact 4260

ttccacaccc taactgacac acattccaca gctggttctt tccgcctcag gactcttcct 4320

ttttcaataa atcaatctaa agtatatatg agtaaacttg gtctgacagt taccaatgct 4380

taatcagtga ggcacctatc tcagcgatct gtctatttcg ttcatccata gttgcctgac 4440

tccccgtcgt gtagataact acgatacggg agggcttacc atctggcccc agtgctgcaa 4500

tgataccgcg agacccacgc tcaccggctc cagatttatc agcaataaac cagccagccg 4560

gaagggccga gcgcagaagt ggtcctgcaa ctttatccgc ctccatccag tctattaatt 4620

gttgccggga agctagagta agtagttcgc cagttaatag tttgcgcaac gttgttgcca 4680

ttgctacagg catcgtggtg tcacgctcgt cgtttggtat ggcttcattc agctccggtt 4740

cccaacgatc aaggcgagtt acatgatccc ccatgttgtg caaaaaagcg gttagctcct 4800

tcggtcctcc gatcgttgtc agaagtaagt tggccgcagt gttatcactc atggttatgg 4860

cagcactgca taattctctt actgtcatgc catccgtaag atgcttttct gtgactggtg 4920

agtactcaac caagtcattc tgagaatagt gtatgcggcg accgagttgc tcttgcccgg 4980

cgtcaatacg ggataatacc gcgccacata gcagaacttt aaaagtgctc atcattggaa 5040

aacgttcttc ggggcgaaaa ctctcaagga tcttaccgct gttgagatcc agttcgatgt 5100

aacccactcg tgcacccaac tgatcttcag catcttttac tttcaccagc gtttctgggt 5160

gagcaaaaac aggaaggcaa aatgccgcaa aaaagggaat aagggcgaca cggaaatgtt 5220

gaatactcat actcttcctt tttcaatatt attgaagcat ttatcagggt tattgtctca 5280

tgagcggata catatttgaa tgtatttaga aaaataaaca aataggggtt ccgcgcacat 5340

ttccccgaaa agtgccacct gacgcgccct gtagcggcgc attaagcgcg gcgggtgtgg 5400

tggttacgcg cagcgtgacc gctacacttg ccagcgccct agcgcccgct cctttcgctt 5460

tcttcccttc ctttctcgcc acgttcgccg gctttccccg tcaagctcta aatcgggggc 5520

tccctttagg gttccgattt agtgctttac ggcacctcga ccccaaaaaa cttgattagg 5580

gtgatggttc acgtagtggg ccatcgccct gatagacggt ttttcgccct ttgacgttgg 5640

agtccacgtt ctttaatagt ggactcttgt tccaaactgg aacaacactc aaccctatct 5700

cggtctattc ttttgattta taagggattt tgccgatttc ggcctattgg ttaaaaaatg 5760

agctgattta acaaaaattt aacgcgaatt ttaacaaaat attaacgct 5809

<210> 18
<211> 275
<212> PRT
<213> artificial

<220>
<223> PRRSV lelystad GP3 fused to HA-tag

<400> 18

Met Ala His Gln Cys Ala Arg Phe His Phe Phe Leu Cys Gly Phe Ile
1               5                   10                  15

Cys Tyr Leu Val His Ser Ala Leu Ala Ser Asn Ser Ser Ser Thr Leu
            20                  25                  30

Cys Phe Trp Phe Pro Leu Ala His Gly Asn Thr Ser Phe Glu Leu Thr
        35                  40                  45

Ile Asn Tyr Thr Ile Cys Met Pro Cys Ser Thr Ser Gln Ala Ala Arg
    50                  55                  60

Gln Arg Leu Glu Pro Gly Arg Asn Met Trp Cys Lys Ile Gly Tyr Asp
65                  70                  75                  80

Arg Cys Glu Glu Arg Asp His Asp Glu Leu Leu Met Pro Ile Pro Ser
                85                  90                  95

Gly Tyr Asp Asn Leu Lys Leu Glu Gly Tyr Tyr Ala Trp Leu Ala Phe
            100                 105                 110

Leu Ser Phe Ser Tyr Ala Ala Gln Phe His Pro Glu Leu Phe Gly Ile
        115                 120                 125

Gly Asn Val Ser Arg Val Phe Val Asp Lys Arg His Gln Phe Ile Cys
    130                 135                 140

Ala Glu His Asp Gly Gln Asn Ser Thr Val Ser Thr Gly His Asn Ile
145                 150                 155                 160

Ser Ala Leu Tyr Ala Ala Tyr Tyr His His Gln Ile Asp Gly Gly Asn
                165                 170                 175

Trp Phe His Leu Glu Trp Leu Arg Pro Leu Phe Ser Ser Trp Leu Val
            180                 185                 190

Leu Asn Ile Ser Trp Phe Leu Arg Arg Ser Pro Val Ser Pro Val Ser
        195                 200                 205

Arg Arg Ile Tyr Gln Ile Leu Arg Pro Thr Arg Pro Arg Leu Pro Val
    210                 215                 220

Ser Trp Ser Phe Arg Thr Ser Ile Val Ser Asp Leu Thr Gly Ser Gln
225                 230                 235                 240

Gln Arg Lys Arg Lys Phe Pro Ser Glu Ser Arg Pro Asn Val Val Lys
                245                 250                 255

Pro Ser Val Leu Pro Ser Thr Ser Arg Ala Tyr Pro Tyr Asp Val Pro
            260                 265                 270

Asp Tyr Ala
        275

## Description

### FIELD OF THE INVENTION

The present invention relates to the field of arteriviruses. In particular, the present invention discloses a method for the production of virus-like particles comprising an arterivirus glycoprotein (GP) called a GP₅ protein. The present invention additionally discloses such virus-like particles for use as medicaments, including for vaccination.

### BACKGROUND OF THE INVENTION

Arteriviruses are responsible for various diseases occurring in livestock. The family of arteriviridae viruses comprises four enveloped, positive-stranded RNA viruses: equine arteritis virus, murine lactate dehydrogenate elevating virus, porcine reproductive and respiratory syndrome virus and the simian hemorrhagic fever virus.

Porcine reproductive and respiratory syndrome virus (PRRSV), also known as Blue-Ear Pig Disease, is of particular importance because it causes a disease in pigs, called porcine reproductive and respiratory syndrome (PRRS). This often devastating, pandemic disease causes massive reproductive failure in breeding stock and respiratory tract illness in young pigs. Initially referred to as "mystery swine disease" and "mystery reproductive syndrome," this disease was first reported in 1987 in North America and Central Europe. PRRS is perhaps the economically most important pig disease in the swine industry, often incurring substantial monetary losses, for example, in the U.S., losses average about € 460 million annually.

While vaccination strategies for PRRS and the other diseases caused by the arteriviruses are regularly employed to thwart the virus, the vaccine situation is not optimal. Presently, attenuated live vaccines are utilized; however, the reversion to virulence of these strains has been frequently reported. Furthermore, the shedding of the virus to unvaccinated animals poses a problem. In addition, the attenuated live vaccines are not capable of differentiating between vaccinated and infected pigs.

Therefore, there exists a need for improved treatments, especially vaccine-based treatments, against infections caused by arteriviridae.

### SUMMARY OF THE INVENTION

It is an object of the present invention to provide a virus-like particle comprising an arterivirus protein that can be effectively used as a vaccine against infections caused by arteriviridae. It is a further object of the present invention to provide a vaccine that can distinguish between a vaccinated animal from an animal infected with the wild-type arteriviridae virus.

This object is achieved by a method for the production of a virus-like particle comprising an arterivirus full-length GP₅ protein and/or an arterivirus GP₅ protein having a modified C-terminus comprising the steps of a) transfecting a mammalian cell with a nucleic acid coding for said full-length and/or modified GP₅ protein; and b) culturing the cell. The object of the invention is furthermore achieved by a virus-like particle, a cell culture supernatant, an ultracentrifuge (UC) pellet and a vaccine.

The present invention is based on the unexpected finding that the GP₅ protein is secreted by mammalian cells and is present in the form of a virus-like particle, and that such virus-like particles are major targets of a protective immune response. Accordingly, an object of the invention is to transfect a mammalian cell with a nucleic acid coding for the GP₅ protein, and to provide virus-like particles that will be present in the supernatant of the cell culture which can be used to induce an immune response. Surprisingly, it has also been found that the supernatant of such transfected mammalian cells can directly be used as a vaccine, with no further manipulation required for administration. This result entails various advantages, for example, low production costs and extreme ease in use and handling.

Whenever reference is made to a "GP₅ protein" both the full-length arterivirus GP₅ protein and the arterivirus GP₅ protein with a modified C-terminus are referred to, where not explicitly stated otherwise. Furthermore, the term "virus-like particle" as used herein, refers to a structure having a high molecular weight that is secreted from a cell into the culture medium and is found in the pellet following ultracentrifugation of this same medium. Preferably, "high molecular weight" means a structure having a higher molecular weight form of a GP₅ monomer, i.e. more than about 30 kDa, but with a lower molecular weight than a native virion, i.e. less than about 60 MDa. Even more preferably, "high molecular weight" means a structure having a molecular weight of about 100 kDa to about 10 MDa. In a sucrose gradient centrifugation, the virus-like particle structure is found in similar fractions as the native virion. The term "particle" is used herein synonymously with the term "virus-like particle".

In one embodiment of the invention, the method further comprises the step of isolating the cell culture supernatant comprising the virus like particle. This isolating step has the advantage that said isolated cell culture supernatant can be used as a vaccine against an arterivirus infection, as it has been unexpectedly observed that the supernatant of a mammalian cell transfected with a nucleic acid coding for a GP₅ protein can be used for purposes of immunization.

In another embodiment of the invention, the method further comprises the step of pelleting the virus-like particle comprised in the isolated cell culture supernatant. In a preferred embodiment, this is achieved by subjecting the isolated supernatant to an ultracentrifugation step to provide a pellet (called a UC pellet) comprising the virus like particle. This pelleting step has the advantage that the isolated UC pellet can also be used as a vaccine against an arterivirus infection.

In another embodiment of the invention, the arterivirus GP₅ protein is selected from the group consisting of the GP₅ protein of equine arteritis virus (EAV), the GP₅ protein of lactate dehydrogenate elevating virus (LDV), the GP₅ protein of porcine reproductive and respiratory syndrome virus (PRRSV) and/or the GP₅ protein of simian hemorrhagic fever virus (SHFV).

Preferably, the arterivirus GP₅ protein is the GP₅ protein of EAV and/or the GP₅ protein of PRRSV; even more preferably the arterivirus GP₅ protein is the GP₅ protein of the European PRRSV strain Lelystad (Genbank accession number: M96262) or the US PRRSV strain VR-2332 (Genbank accession number: EF536003).

The full-length GP₅ protein represents the complete arterivirus GP₅ protein, the sequence of which is known to the skilled person. The full-length amino acid sequence of GP₅ derived from the European PRRSV strain Lelystad, the US PRRSV strain VR-2332 and EAV is shown in SEQ ID NOs 1 - 3, respectively.

The term "modified GP₅ protein" or "GP₅ protein with a modified C-terminus" refers to any arterivirus GP₅ protein with a C-terminus that has been altered by addition of amino acids. In specific embodiments of the invention, the modification of the C-terminus is carried out with an addition of ≥ 5 and ≤ 50 amino acids, preferably with ≥ 9 and ≤ 50 amino acids.

In further preferred embodiments of the invention, the C-terminal modification is a marker protein and/or a marker tag. This has the advantage that by means of the marker protein/tag, animals having been vaccinated with a virus-like particle comprising such a modified GP₅ protein can easily be distinguished from animals that were subject to a "real", i.e. wild-type, arterivirus infection (known as the "Diva-concept"). Most preferably, the marker tag is the HA-tag or the FLAG-tag. However, any marker that allows the detection of specific antibodies raised against the marker is suitable for the present invention. Thus, any other immunogenic polypeptide can likewise be used, such as an epitope originating from a different virus, such as a neutralizing epitope of PCV-2 as a preferred example. Particularly preferred embodiments of the invention are set forth in SEQ ID NOs 4, 5 and 6, i.e. the GP₅ of the European and US strains of PRRSV as well as the EAV GP₅ modified by a HA-tag, respectively. In further embodiments, functional homologues of the arterivirus GP₅ proteins are additionally encompassed by the present invention. Preferably, a functional homologue of a given GP₅ protein comprises any amino acid sequence, i.e. a peptide, which upon expression in a mammalian cell will be secreted by a virus-like particle into the cell culture supernatant, and can be used as vaccine against arterivirus infections. As described in the Examples, the skilled person can readily determine whether a given protein acts as a functional homologue of an arterivirus GP₅ protein, i.e. whether an immune response is induced. More preferably, a functional homologue and/or a homologue of a given arterivirus GP₅ protein is any protein that has an amino acid sequence that is ≥ 50 %, ≥ 60 %, ≥ 70 %, ≥ 80 %, ≥ 85 %, or ≥ 90 % identical to the amino acid sequence of the arterivirus protein. Even more preferably, the protein has an amino acid sequence that is ≥ 95 %, ≥ 97 %, ≥ 98 %, ≥ 99 %, ≥ 99.7 %, or ≥ 99.9 %, identical to the amino acid sequence of the arterivirus protein.

The mammalian cell is any cell suitable for producing the virus-like particles according to the present invention. A preferred example for such a mammalian cell is a hamster cell, a pig cell, a horse cell, a monkey cell, more preferably a Vero E6 cell, a Marc145 cell, a HeLa cell, a RK13 cell, a APH-R cell (Heinrich A, et al. Antiviral Res. 2009; 81:209-16), a PK15 cell (Li Wei and Jue Liu, Virology 2009; 386:203-209), and most preferably a BHK21 cell.

Transfection is a process of introducing a nucleic acid into a cell. Transfection of animal cells typically involves opening transient pores in the cell plasma membrane, thus allowing the uptake of the nucleic acids such as plasmid DNA or siRNA constructs. In addition to electro-poration, transfection can be carried out using chemicals, e.g. calcium phosphate, or by mixing a cationic lipid with the material to produce liposomes, which fuse with the cell plasma membrane in order to deposit their cargo inside. The transfection of the mammalian cell can be carried out according to any technique known to the skilled person, for example transfection carried out using lipofectamine.

The method according to the present also provides virus-like particles comprising full-length and modified GP₅ proteins, i.e. a mixture of said GP₅ proteins. Thus, in order to achieve such a mixture a cell may be transfected twice, simultaneously or subsequently, with nucleic acids coding for full-length and with sequences coding for modified GP₅ proteins. However, two single transfections of two different cells with each of the two nucleic acids coding for the respective full-length and modified GP₅ proteins is also possible. Such cells may then be cultured in the same medium which will, in turn include virus-like particles comprising full-length and modified GP5 proteins, respectively. Alternatively, cultivation in separate mediums is possible with a subsequent mixing of the virus-like particles that are produced.

The culturing of the transfected cell can be carried out according to any method known to the skilled person and suitable for the particular cell type. Preferably, the cell is cultured under conditions sufficient to allow for the secretion of virus-like particles according to the present invention. More preferably and when the cell is a BHK21 cell, the culturing is carried out under the following conditions: Growth medium: Glasgow minimum essential medium, supplemented with 10% fetal calf serum, 1% Glutamax, and 5% tryptose phosphate broth.

The isolation of the cell culture supernatant can be carried out using known methodology. In specific embodiments, the isolation of the cell culture supernatant is achieved by decantation, aspiration or pipetting of the supernatant. In a preferred embodiment, a low-speed centrifugation step is carried out before the isolation step. More preferably, low-speed centrifugation means a centrifugation with less than 18.000 x g. In another preferred embodiment, the isolated supernatant is subsequently filtered to remove cell debris; more preferably by means of a 0.2 µm filter unit.

Cell culture supernatant isolated according to these conditions can be directly used for vaccination purposes.

The virus-like particles present in the isolated cell culture supernatant can be pelleted, preferably by means of ultracentrifugation, for example, using a Sorvall Surespin 630 rotor. In a preferred embodiment, the ultracentrifugation is carried out at about 100,000 x g. In a further preferred embodiment, the ultracentrifugation is carried out for about 2 hrs at 4° C. More preferably, the supernatant is filtered prior to subjecting it to the pelleting step, e.g. by means of a filter unit.

The pelleted virus-like particles isolated in such ways can directly be used for vaccination purposes.

In another aspect, the present invention is directed to a virus-like particle comprising an arterivirus full-length GP₅ protein and/or an arterivirus GP₅ protein with a modified C-terminus or a functional homologue thereof. In a preferred embodiment, the virus-like particle is produced by means according to the method of the present invention.

In yet another aspect, the present invention is directed to a cell culture supernatant comprising a virus-like particle according to the invention, i.e. a virus-like particle comprising an arterivirus full-length GP₅ protein and/or an arterivirus GP₅ protein with a modified C-terminus or a functional homologue thereof. In a preferred embodiment, the virus-like particle is produced by means according to the method of the present invention. In another preferred embodiment of the invention, the cell culture supernatant further comprises cellular components from a mammalian cell. Preferably such components are cell organelles and/or remnants thereof, cytosolic components, and/or nucleic acids.

In a further aspect, the present invention is directed to a pellet comprising a virus-like particle according to the invention, i.e. a virus-like particle comprising an arterivirus full-length GP₅ protein and/or an arterivirus GP₅ protein with a modified C-terminus or a functional homologue thereof. In a preferred embodiment, the pellet is produced by means according to the method of the present invention. In another preferred embodiment of the invention, the pellet is a UC pellet as described previously.

In a further aspect, the present invention is directed to a vaccine comprising a virus-like particle according to the invention, i.e. a virus-like particle comprising an arterivirus full-length GP₅ protein and/or an arterivirus GP₅ protein with a modified C-terminus or a functional homologue thereof.

In a preferred embodiment, the vaccine further comprises an adjuvant. The adjuvant can be any adjuvant known to the skilled person. In a preferred embodiment, the adjuvant is selected from the group consisting of Freuds adjuvant, Aluminium hydroxide, paraffin, carbomers, and/or oil-in-water emulsions.

In a further aspect, the present invention is directed to the use of the virus-like particle, the cell culture supernatant, the pellet and/or the vaccine according to the invention for the preparation of a medicament.

In another aspect, the present invention encompasses the use of the virus-like particle, the cell culture supernatant the pellet and/or the vaccine according to the invention for the preparation of a medicament for the treatment and/or prevention of an arterivirus infection, preferably of an infection with EAV, PRRSV, LDV and/or SHFV. In further preferred embodiments, the prepared medicament is used for the treatment and/or prevention of lethal hemorrhagic fever, necrosis of the small muscular arteries, respiratory infections, abortions, and/or still birth.

In further specific embodiments of the invention, the presently described vaccines or medicaments can be used to treat and/or vaccinate an animal, preferably a pig against PRRSV, a horse against EAV, a mouse against LDV and/or a monkey against SHFV.

The various embodiments of the virus-like particle, the cell culture supernatant the pellet, the vaccine and/or the medicament according to the invention correspond to those detailed above regarding the present methods. However, in the most preferred embodiment of the invention, the arterivirus GP₅ protein is the GP₅ protein of EAV and/or the GP₅ protein of the European PRRSV strain Lelystad or the US PRRSV strain VR-2332 or a functional homologue thereof.

Further advantageous embodiments of the present invention are described herein. These and other aspects of the invention will be apparent from and elucidated with reference to the instant embodiments.

### BRIEF DESCRIPTION OF THE DRAWINGS

- FIG. 1: A Western blot analysis of the supernatant of GP₅_HA transfected cell lines using an anti-HA antibody.
- FIG. 2A: A Western blot analyses of supernatant and ultracentrifugation pellet of BHK21 cells transfected with GP₅_lely_pT (top panel), VR_GP₅_HA (middle panel) and GP₃_HA (bottom panel), respectively. "Cells" refers to the cell lysate; "supernatant" is the filtered supernatant; "pellet 100 000 g" is the UC-pellet material.
- FIG. 2B: A Western blot analyses of supernatant of BHK21 cells transfected with GP₅_HA (top panel, anti HA-antibody) after low-speed centrifugation compared to native PRRSV virions (bottom panel, anti- GP₅ serum).
- FIG. 2C: A Western blot analysis of cell culture supernatant before and after ultracentrifugation.
- FIG. 3: A Western blot analyses of the supernatant of BHK cells transfected with GP₅_lely_pT and native PRRSV virions (European strain) after sucrose gradient centrifugation. The fractions were analyzed using anti_ GP₅ serum.
- FIG. 4: An electron microscopic image of UC-pellet material obtained from BHK21 cells transfected with GP₅_HA (left) and pQE (right).
- FIG. 5: A Western blot analyses of sera of mice injected with UC-pellet material or supernatant of GP₅_HA transfected BHK21 cells. The positions of the antigens are indicated by arrows.
- FIG. 6: Results following a virus neutralization test.

### DETAILED DESCRIPTION OF THE INVENTION

The arteriviridae family comprises four enveloped, positive-stranded 3'-polyadenylated RNA viruses: the equine arteritis virus, murine lactate dehydrogenate elevating virus, porcine reproductive and respiratory syndrome virus and the simian hemorrhagic fever virus.

Arteriviruses are typically spherical, approximately 40 to 60 nm in diameter, and normally have a relatively smooth surface that lacks large projections. They consist of an isometric, probably icosahedral, nucleocapsid of about 25 to 35 nm, which is surrounded by an envelope carrying small projections. The buoyant density of arteriviruses is about 1.13 to 1.17 g per/cm³ in sucrose and their sedimentation coefficient ranges from 214S to 230S.

The arterivirus nucleocapsid comprises an RNA genome of about 12 to 16 kb and the nucleocapsid protein (N). Based on studies involving EAV and PRRSV, the lipid bilayer surrounding the nucleocapsid most likely comprises six envelope proteins: M, E, GP₂, GP₃, GP₄, and GP₅, wherein the major envelope proteins M and GP₅ form a disulfide-linked heterodimer and the minor glycoproteins GP₂, GP₃ and GP₄ form a disulfide-linked heterotrimer, as well as GP₂ - GP₄ dimers (identified in EAV strains). It is noted that in SHFV the protein corresponding to GP₅ of the remaining arteriviruses is actually called GP₇. However, for the sake of convenience said SHFV GP₇ (also known as p54) is likewise referred to as GP₅.

The GP₅ proteins of arteriviruses share distinct common structural features. These proteins contain an N-terminal signal sequence that is thought to be cleaved from a short ectodomain. The central hydrophobic region is assumed to span the membrane three times and to be followed by a cytoplasmic domain of about 50 to 75 amino acids. In case of the LDV and PRRSV strains, the ectodomain is only about 30 amino acid residues in length and contains one to three N-linked glycans.

The clinical features and pathogenesis of EAV and PRRSV can be summarized as follows. An EAV infection usually results in subclinical to flulike symptoms in adult animals, abortion in pregnant mares and interstitial pneumonia in neonates. Clinical features are characteristic vascular lesions, necrosis of small muscular arteries, acute anorexia and fever, usually accompanied by palpebral edema, conjunctivitis, nasal catarrh and edema of the legs, genitals and abdomen. A PRRSV infection usually results in fever, anorexia, labor respiration, lymphadenopathy, gross and microscopic lesions in the lung and reproductive failure characterized by delivery of weak or stillborn piglets, or autolysed fetuses.

The present invention is based on the unexpected finding that the arterivirus GP₅ protein is secreted into the culturing medium by mammalian cells in the form of a virus-like particle. Surprisingly, such virus-like particles function as major targets in a protective immune response and therefore the virus-like particles can be used to effectively induce an immune response. Furthermore, it has also been found that the crude supernatant, i.e. not requiring any purification and/or concentration steps, from mammalian cells secreting the arterivirus GP₅ protein and/or the virus-like particles comprising said protein, can be directly used as a vaccine.

The present invention provides a method for the production of such virus-like particles. In a specifically preferred embodiment, a virus-like particle comprising a EAV or PRRSV arterivirus full-length GP₅ protein and/or a GP₅ protein C-terminally modified by a marker tag, preferably the HA- or FLAG-tag, is produced by transfecting a BHK21 cell with a nucleic acid coding for said full-length and/or a modified GP₅ protein; culturing the cell; and isolating the cell culture supernatant comprising said virus like particle. Cell culture supernatant isolated in this way can directly be used as a vaccine, preferably in combination with Freuds adjuvant. Additionally, however, the supernatant can be subjected to an addition pelleting step of the virus-like particle comprised in the isolated cell culture supernatant, preferably by means of an ultracentrifugation step at about 100,000 x g for about 2 hrs at 4° C. The UC pellet thus generated can likewise be used as a vaccine, preferably in combination with Freuds adjuvant.

Advantageously, the vaccine created by the method of the invention comprises the full-length GP₅ protein as well as the GP₅ protein C-terminally modified by the described marker tag. Therefore, animals vaccinated with the vaccine of the invention can easily be distinguished from animals that were subject to a "real", i.e. wild-type, arterivirus infection ("Diva-concept").

### EXAMPLES

### Example 1: Generation of Plasmid Constructs

The plasmids GP₅_lely_pT (SEQ ID NO: 7) and VR_ GP₅ (SEQ ID NO: 8) were constructed by inserting the gene for GP₅ of the European lelystad and USA VR-2332 strain of PRSSV, respectively, into the multiple cloning site of pCR3.1 (Invitrogen). The cloning was carried out using a BamHI and XhoI restriction digest. The HA-tag, corresponding to amino acids 98-106 of Human influenza hemagglutinin (Genbank accession no: AAB02235) was added to the last C-terminal amino acid of GP₅ by PCR, yielding the constructs GP₅_HA (European strain; SEQ ID NO: 9) and VR_ GP₅_HA (US-strain; SEQ ID NO: 10). A single alanine residue was introduced as a spacer between GP₅ and the HA-tag.

Likewise, plasmids EAV_GP₅ (SEQ ID NO: 11) and EAV_GP₅_HA (SEQ ID NO: 12) of the EAV GP₅ were constructed, as well as the plasmids EAV_GP₅ _FLAG (SEQ ID NO: 13), i.e. the EAV GP₅ protein fused to a FLAG-tag, and GP₅_lely_PCV2 (SEQ ID NO: 14), i.e. the GP₅ of the European lelystad strain fused to the PCV2 neutralizing epitope. In addition, a plasmid comprising the HA-tagged GP₃ of the European lelystad strain (GP3_HA; SEQ ID NO: 17) was constructed.

All plasmids were verified by DNA sequencing.

The plasmids thus code for the following amino acid sequences:
GP₅ of the European strain, SEQ ID NO: 1: 
M R C S H K L G R F L T P H S C F W W F F L L C T G L S W S F A D G N G N S S T Y
Q Y I Y N L T I C E L N G T D W L S S H F G W A V E T F V F Y P V A T H I L S L G F
L T T S H F F D A L G L G A V S T A G F V G G R Y V L C S V Y G A C A F A A F V C
F V I R A A K N C M A C R Y A R T R F T N F I V D N R G R V H R W K S P I V V E K
L G K A E V D G N L V T I K H V V L E G V K A Q P L T R T S A E Q W E A
GP₅ of the European strain fused to HA-tag (underlined), SEQ ID NO: 4: 
M R C S H K L G R F L T P H S C F W W F F L L C T G L S W S F A D G N G N S S T Y
Q Y I Y N L T I C E L N G T D W L S S H F G W A V E T F V F Y P V A T H I L S L G F
L T T S H F F D A L G L G A V S T A G F V G G R Y V L C S V Y G A C A F A A F V C
F V I R A A K N C M A C R Y A R T R F T N F I V D N R G R V H R W K S P I V V E K
L G K A E V D G N L V T I K H V V L E G V K A Q P L T R T S A E Q W E A A Y P Y D
V P D Y A
GP₅ of the European strain fused to the PCV2 neutralizing epitope (underlined), SEQ ID NO: 15: 
M R C S H K L G R F L T P H S C F W W F F L L C T G L S W S F A D G N G N S S T Y
Q Y I Y N L T I C E L N G T D W L S S H F G W A V E T F V F Y P V A T H I L S L G F
L T T S H F F D A L G L G A V S T A G F V G G R Y V L C S V Y G A C A F A A F V C
F V I R A A K N C M A C R Y A R T R F T N F I V D N R G R V H R W K S P I V V E K
L G K A E V D G N L V T I K H V V L E G V K A Q P L T R T S A E Q W E A A G S G A
K P V L D S T I D Y F Q P N N K R N Q L W L R L Q T A G N V D H V G L G
GP₅ of the US-strain, SEQ ID NO: 2: 
M L E K C L T A G C C S R L L S L W C I V P F C F A V L A N A S N D S S S H L Q L I
Y N L T L C E L N G T D W L A N K F D W A V E S F V I F P V L T H I V S Y G A L T T
S H F L D T V A L V T V S T A G F V H G R Y V L S S I Y A V C A L A A L T C F V I R
 
F A K N C M S W R Y A C T R Y T N F L L D T K G R L Y R W R S P V I I E K R G K V
E V E G H L I D L K R V V L D G S V A T P I T R V S A E Q W G R P
GP₅ of the US-strain fused to HA-tag (underlined), SEQ ID NO: 5: 
M L E K C L T A G C C S R L L S L W C I V P F C F A V L A N A S N D S S S H L Q L I
Y N L T L C E L N G T D W L A N K F D W A V E S F V I F P V L T H I V S Y G A L T T
S H F L D T V A L V T V S T A G F V H G R Y V L S S I Y A V C A L A A L T C F V I R
F A K N C M S W R Y A C T R Y T N F L L D T K G R L Y R W R S P V I I E K R G K V
E V E G H L I D L K R V V L D G S V A T P I T R V S A E Q W G R P A Y P Y D V P D
Y A
GP₅ of EAV, SEQ ID NO: 3: 
M L S M I V L L F L L W G A P S H A Y F S Y Y T A Q R F T D F T L C M L T D R G V
I A N L L R Y D E H T A L Y N C S A S K T C W Y C T F L D E Q I I T F G T D C N D T
Y A V P V A E V L E Q A H G P Y S V L F N D M P P F I Y Y G R E F G I V V L D V F
M F Y P V L V L F F L S V L P Y A T L I L E M C V S I L F I I Y G I Y S G A Y L A M G
I F A A T L A I H S I V V L R Q L L W L C L A W R Y R C T L H A S F I S A E G K V Y
P V D P G L P V A A A G N R L L V P G R P T I D Y A V A Y G S K V N L V R L G A A
E V W E P
GP₅ of EAV fused to HA-tag (underlined), SEQ ID NO: 6: 
MLSMIVLLFLLWGAPSHAYFSYYTAQRFTDFTLCMLTDRGVIANLLRYDE
HTALYNCSASKTCWYCTFLDEQIITFGTDCNDTYAVPVAEVLEQAHGPYS
VLFNDMPPFIYYGREFGIVVLDVFMFYPVLVLFFLSVLPYATLILEMCVS
ILFIIYGIYSGAYLAMGIFAATLAIHSIVVLRQLLWLCLAWRYRCTLHAS
FISAEGKVYPVDPGLPVAAAGNRLLVPGRPTIDYAVAYGSKVNLVRLGAA
EVWEPAYPYDVPDYA
GP₅ of EAV fused to FLAG-tag (underlined), SEQ ID NO: 16: MLSMIVLLFLLWGAPSHAYFSYYTAQRFTDFTLCMLTDRGVIANLLRYDE 
HTALYNCSASKTCWYCTFLDEQIITFGTDCNDTYAVPVAEVLEQAHGPYS
VLFNDMPPFIYYGREFGIVVLDVFMFYPVLVLFFLSVLPYATLILEMCVS
ILFIIYGIYSGAYLAMGIFAATLAIHSIVVLRQLLWLCLAWRYRCTLHAS
FISAEGKVYPVDPGLPVAAAGNRLLVPGRPTIDYAVAYGSKVNLVRLGAA
EVWEPADYKDDDDK
GP₃ of European strain fused to HA-tag (underlined), SEQ ID NO: 18:

MAHQCARFHFFLCGFICYLVHSALASNSSSTLCFWFPLAHGNTSFELTIN
YTICMPCSTSQAARQRLEPGRNMWCKIGYDRCEERDHDELLMPIPSGYDN
LKLEGYYAWLAFLSFSYAAQFHPELFGIGNVSRVFVDKRHQFICAEHDGQ
NSTVSTGHNISALYAAYYHHQIDGGNWFHLEWLRPLFSSWLVLNISWFLR
RSPVSPVSRRIYQILRPTRPRLPVSWSFRTSIVSDLTGSQQRKRKFPSES
RPNVVKPSVLPSTSRAYPYDVPDYA

### Example 2: Transfection and Harvesting of the GP5 Virus-Like Particles

5 x 10⁵ BHK21 cells were seeded in small culture flasks and transfected with 8 µg of the DNA constructs. Transfection was carried out with Lipofectamine 2000 (Invitrogen) according to the manufacturer's instructions. After 8 hours the transfection medium was removed and replaced by normal culture medium.

The transfection assay was successfully repeated using Vero E6 cells.

42 hours after transfection, the supernatant was removed from the cells and filtered through a 0.2 µm filter unit. The supernatant was stored at -80°C until it was used for vaccination or in an SDS PAGE protocol.

Alternatively, for biochemical characterization of the particles, the supernatant was filtered and subjected to ultracentrifugation (UC) at 100 000 x g for 2 hours at 4°C in a Sorvall Surespin 630 rotor. The pellet was resuspended in PBS and analyzed by a 5% - 40% sucrose gradient centrifugation, by electron microscopy (both according to Vennema et al., 1996. EMBO J. 15) or directly by SDS PAGE. The proteins were identified in a Western blot by antibodies raised in rabbits against the peptide AQPLTRTSAEQWEA or by a monoclonal anti-HA antibody (Millipore).

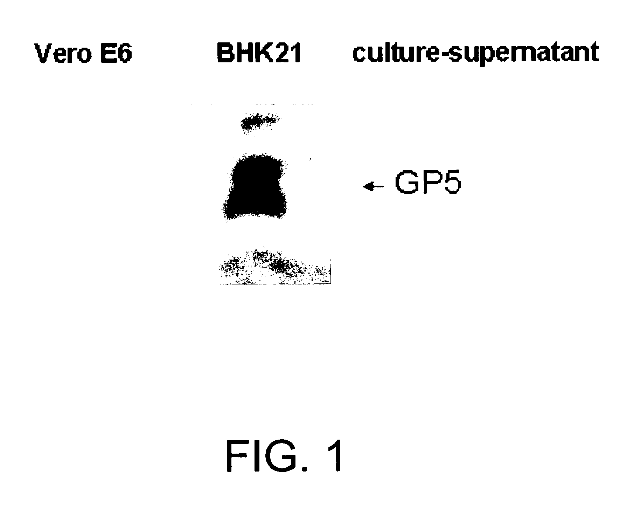

FIG. 1 shows the results of the transfection of Vero E6 cells and BHK21 cells with GP₅-HA. Two days after transfection, the supernatant was removed and analyzed by Western blot using the anti-HA antibody. While the HA-tagged GP₅ protein is clearly secreted into the supernatant of Vero E6 cells, BHK21 clearly showed substantially more GP₅ protein secretion. Therefore, subsequent experiments were carried out using the BHK21 cells.

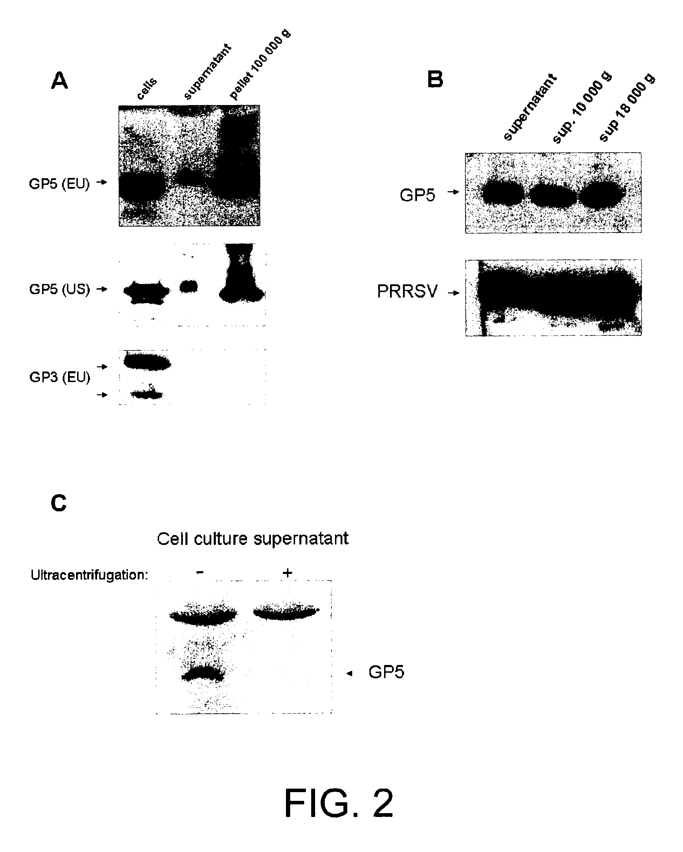

FIG. 2A shows the results of transfection experiments in BHK21 cells using the plasmids of the present invention. FIG. 2A shows the transfection with GP₅_lely_{_}pT (top panel), VR_GP₅_HA (middle panel) and GP₃_HA as a control (bottom panel), respectively. Two days after transfection, the supernatant was removed and subjected to ultracentrifugation. The cells were lysed and all samples were analyzed by Western blot using an anti-GP₅ serum (top panel) and an anti-HA antibody (middle and bottom panels). "Cells" means the cell lysate; "supernatant" is the filtered supernatant; "pellet 100 000 g" is the UC-pellet material. Clearly, the secreted GP₅ of both the European and the American PRRSV strain is present in the cells, the supernatant and the pellet, while GP₃ is only present in the cells, and is thus not secreted into the culturing medium.

FIG. 2B shows the results of the transfection of BHK21 cells with GP₅_HA. Two days after transfection the supernatant was removed, subjected to the indicated centrifugation steps and analyzed by Western blot (top panel, anti HA-antibody). Native PRRSV virions were used as a control (bottom panel, anti-GP₅ serum). The secreted GP₅ protein is shown to be full length compared to the native protein (Matanin *et al*., 2008. J. Virol. Meth. 147). Furthermore, secreted GP₅ is not pelleted by low-speed centrifugation, similar to the native virions. However, as shown in FIG. 2A, the GP₅ is pelleted by ultracentrifugation at 100 000 g, suggesting that secreted GP₅ is present in a form of a virus-like particle.

FIG. 2C shows a Western blot analysis of cell culture supernatant before and after ultracentrifugation. Culture supernatant of BHK21 cells was collected 2 days after transfection with GP₅_HA. The results show that the GP₅ signal is no longer present following the centrifugation step, meaning that all virus-like particles are present in the pellet fraction.

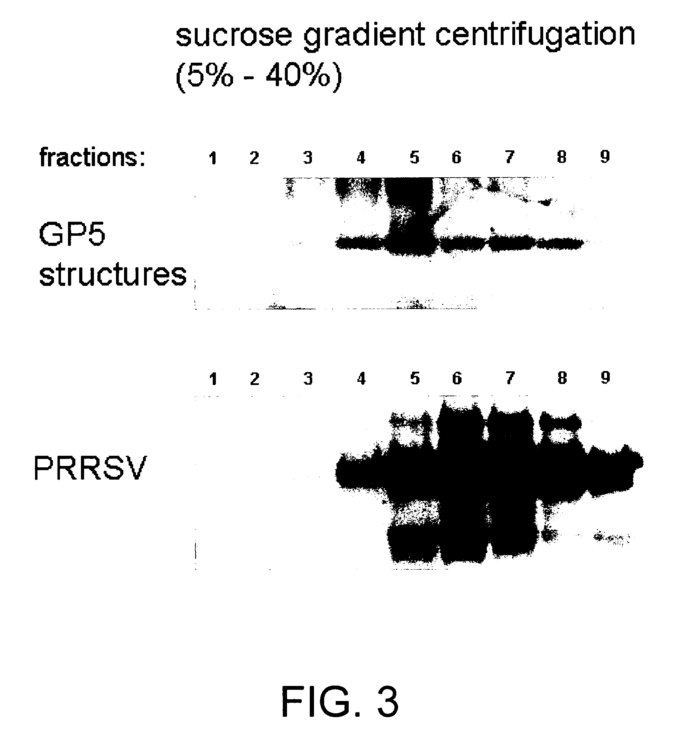

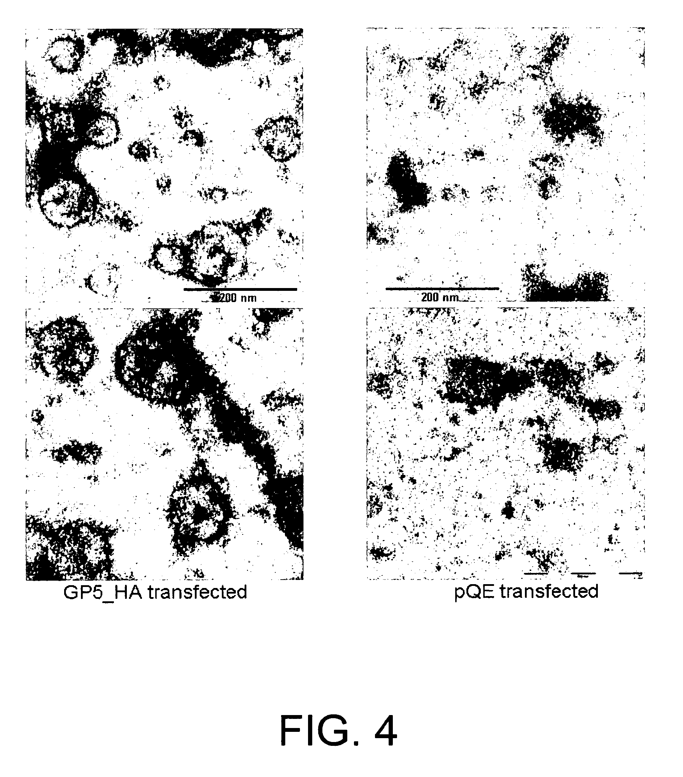

GP₅ secretion in the form of virus-like particles is furthermore supported by the behaviour of secreted GP₅ in a sucrose gradient centrifugation, as shown in FIG. 3. In this experiment, the supernatant of BHK cells transfected with GP₅_lely-pT and the native PRRSV virions (European strain) were subjected to sucrose gradient centrifugation. The fractions were analyzed by Western blot using the anti_GP₅ serum. As can be seen, GP₅ enters the gradient and peaks in a fraction close to the native PRRSV-virions. In addition, electron microscopic analysis of the pellet-material after ultracentrifugation showed the enrichment of roundish particles heterogeneous in size (20-100 nm). By contrast, such particles occurred with much lower frequency in the control samples (i.e. obtained after transfecting an empty pQE plasmid, available from Qiagen). FIG. 4 shows the electron microscopic images of UC-pellet material obtained from BHK21 cells transfected with GP₅_HA (left) and pQE (right).

### Example 3: Animal Vaccination

Both the supernatant and the pellet obtained following ultracentrifugation, according to the methods of the present invention, were used to immunize mice.

The supernatant of GP₅_HA transfected BHK21 cells was filtered and mixed for 30 min with an equal volume of the adjuvant ImjetAlum (Thermo Scientific). 2 mice were injected intraperitoneally with 500 µl (per mouse) of the mixture. 2 mice were injected with 100 µg (per mouse) of UC-pellet material mixed with adjuvant, and 2 mice were injected with 200 µg (per mouse) of UC-pellet material without adjuvant. Animals were boosted with the same doses 2 weeks later. Animals were sacrificed 4 weeks after the first injection. The blood was analyzed by Western blot and VNT. For the Western blot, sera were incubated in a 1/50 dilution with a membrane containing UC-pellet material from BHK21 cells transfected with GP₅_lelypT or GP₅ _HA.

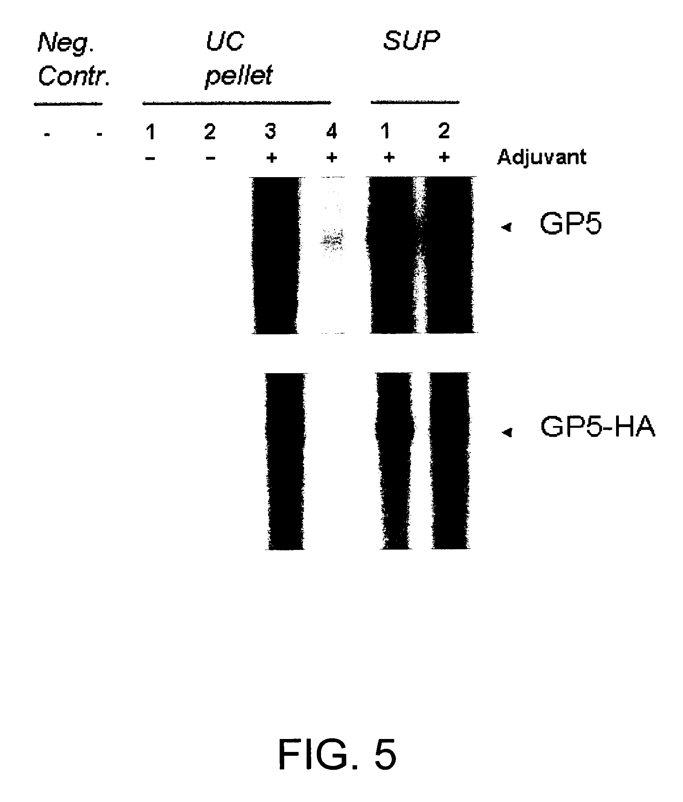

FIG. 5 shows sera of mice injected with UC-pellet material or supernatant of GP₅_HA transfected BHK21 cells. The sera were analyzed by Western blot for their reactivity with GP₅_lely_pT (top panel) or GP₅_HA (bottom panel). The positions of the antigens are indicated by arrows. FIG. 5 clearly reveals that antibodies against the protein were detectable, and most pronounced in the animals immunized directly with the supernatant.

The experiments were repeated with the supernatant and UC-pellet material of EAV_GP₅_FLAG and VR_GP₅ _HA transfected BHK21 and Vero E6 cells, and yielded similar results observed with the GP₅_HA transfected BHK21 cells as described above.

The supernatant and pellets of GP₅ transfected mammalian cells can therefore be used as a vaccine against a viral infection.

### Example 4: Virus neutralization tests (VNT)

To carry out the VNT protocols, Marc145 cells were plated in 96 well plates (1 x 104 cells per well). The following day, mice sera were incubated at 56°C for 30 min to inactivate complement. Subsequently, sera were diluted in 1:1 steps in 200 µl volumes and mixed with 200 µl of medium containing the European DV strain of PRRSV (10³ TCID50 / ml). After incubation at 37°C for one hour, the mixture was added to the cells (100 µl per well). Serum from a vaccinated pig was taken along as a positive control. Virus neutralization was monitored microscopically by determining cytopathic effects 5 days later.

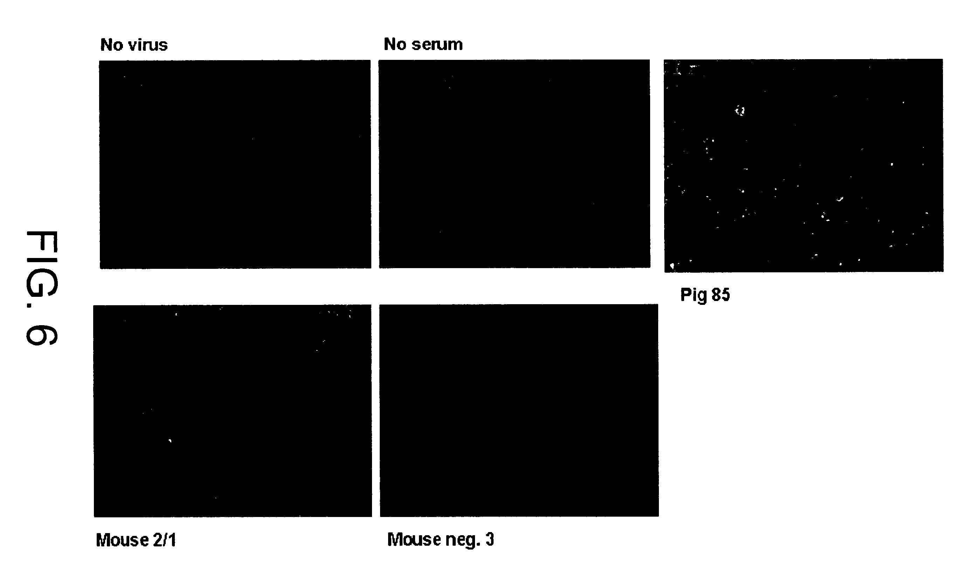

FIG. 6 shows images of Marc145 cells used in the VNT. Sera was derived from a mouse vaccinated with the supernatant derived from the GP₅_HA transfected BHK21 cells ("Mouse 2/1 "), a non-vaccinated mouse ("Mouse neg. 3"), and from a pig vaccinated against the European strain of PRRSV ("Pig 85"). Images of sera dilution 1:4 were taken 5 days after the VNT were initiated. As can be taken from FIG. 6, these sera also contained neutralizing antibodies.

In summary, the supernatant of GP₅ transfected mammalian cells can be used as a vaccine against a viral infection.

While the invention has been illustrated and described in detail in the drawings and foregoing description, such illustration and description are to be considered illustrative or exemplary and not restrictive; the invention is not limited to the disclosed embodiments. Other variations to be disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to achieve the technical effects described herein. Any reference signs in the claims should not be construed as limiting scope.

## Claims

1. A method for the production of a virus-like particle comprising an arterivirus full-length GP₅ protein and/or an arterivirus GP₅ protein with a modified C-terminus or a functional homologue thereof comprising the steps of:
a) transfecting a mammalian cell with a nucleic acid coding for said full-length and/or modified GP₅ protein; and
b) culturing the cell.

2. The method according to claim 1, further comprising the step of:
c) isolating the cell culture supernatant comprising said virus-like particle.

3. The method according to claim 2, further comprising the step of:
d) pelleting the virus-like particle comprised in the isolated cell culture supernatant.

4. The method according to any of the preceding claims, wherein the arterivirus protein GP₅ is selected from the group consisting of the GP₅ protein from EAV, LDV, PRRSV, SHFV, the European PRRSV strain Lelystad and/or from the US PRRSV strain VR-2332.

5. The method according to any of the preceding claims, wherein the modified C-terminal comprises an addition of ≥ 5 and ≤ 50 amino acids, a marker protein, a marker tag, an HA-tag and/or a FLAG-tag.

6. The method according to any of the preceding claims, wherein the mammalian cell is a hamster cell, pig cell, horse cell, monkey cell, a vero E6 cell, APH-R cell, PK15 cell or a BHK21 cell.

7. The method according to claims 3 to 6, wherein the pelleting in step d) is achieved by ultracentrifugation.

8. A virus-like particle comprising an arterivirus full-length GP₅ protein and/or an arterivirus GP₅ protein with a modified C-terminus or a functional homologue thereof.

9. A cell culture supernatant comprising a virus-like particle comprising an arterivirus full-length GP₅ protein and/or an arterivirus GP5 protein with a modified C-terminus or a functional homologue thereof.

10. A pellet comprising virus-like particles comprising an arteriviridae full-length GP₅ protein and/or an arteriviridae GP₅ protein with a modified C-terminus or a functional homologue thereof.

11. The virus-like particle according to claim 8, the cell culture supernatant according to claim 9 or the pellet according to claims 10, wherein the modified C-terminal comprises an addition of ≥ 5 and ≤ 50 amino acids, a marker protein, a marker tag, an HA-tag and/or a FLAG-tag.

12. The virus-like particle according to claim 8, the cell culture supernatant according to claim 9 or the pellet according to claims 10, wherein the mammalian cell is a vero E6 cell or a BHK21 cell.

13. A vaccine comprising the virus-like particle according to claims 8, 11 and 12, the cell culture supernatant according to claims 9, 11 and 12 and/or the pellet according to claims 10, 11 and 12.

14. The virus-like particle according to claims 8, 11 and 12, the cell culture supernatant according to claims 9, 11 and 12 and/or the pellet according to claims 10, 11 and 12 as a medicament.

15. The virus-like particle according to claims 8, 11 and 12, the cell culture supernatant according to claims 9, 11 and 12 and/or the pellet according to claims 10, 11 and 12 as a medicament for the treatment and/or prevention of an arterivirus infection.
